# EUROPEAN PATENT APPLICATION

(11) **EP 2 939 690 A1**
(43) Date of publication of application: **04.11.2015**
(21) Application number: 14186355.5
(22) Date of filing: 25.09.2014
(51) Int. Cl.: A61K 39/00, A61K 39/395

(54) **Vaccine**

(30) Priority: 29.04.2014 EP 14166480
(71) Applicant: Medizinische Hochschule Hannover, 30625 Hannover (DE)
(72) Inventor: Wirth, Dr. Thomas, 31319 Sehnde (DE)
(74) Representative: Taruttis, Stefan Georg

(57) **Abstract**

The present invention relates to a pharmaceutical combination of compositions for use in the treatment or prevention of a disease having cells bearing a target antigen as a vaccine and to a method for vaccination of a mammal, especially of a human for raising a cellular immune response directed against cells of the mammalian recipient, especially human recipient, which cells express a target antigen. The target antigen can e.g. be an autoantigen like a malignant antigen, i.e. a tumour-specific antigen. The pharmaceutical combination of compositions comprises a first composition and a second composition, wherein the second composition is for administration to recipient subsequent to the administration of the first composition, e.g. 2 to 10 days after the first composition. The pharmaceutical combination of compositions has the advantage of raising an effective antigen-specific T-cell response against cells bearing a target antigen that can be a malignant autoantigen, e.g. for raising an antigen-specific T-cell response against cells bearing a tumour-antigen. A further advantage is that the pharmaceutical combination of compositions can raise an antigen-specific T-cell response within a comparatively short time.

## Description

The present invention relates to a pharmaceutical combination of compositions for use in the treatment or prevention of a disease having cells bearing a target antigen, e.g. as a vaccine and to a method for vaccination of a mammal, especially of a human for raising a cellular immune response directed against cells of the mammalian recipient, especially human recipient, which cells express a target antigen. The target antigen can e.g. be an autoantigen like a malignant antigen, i.e. a tumour-specific antigen, or an alloantigen specific for an infecting agent, e.g. an antigen specific for a virus or for an intracellular bacterium. Preferably, the pharmaceutical combination of compositions and the method are for medical use in the treatment of tumour and/or for medical use in the treatment of infections by a virus or by intracellular bacteria.

The pharmaceutical combination of compositions comprises or consists of a first composition and a second composition, wherein the second composition is for administration to the mammalian, especially human recipient, subsequent to the administration of the first composition, e.g. the second composition is provided for administration at least 1 day, preferably 2 to 10 days, e.g. 7 days following administration of the first composition. Accordingly, the method of medical treatment comprises the administration of the first composition and the subsequent administration of the second composition to a recipient, e.g. at least one day, preferably 2 to 10 days subsequent to administration of the first composition. The method of treatment is effective in inducing an antigen-specific T-cell response in the recipient, which response is directed against cells bearing the target antigen, which preferably is an auto-antigen like a tumour-antigen. The pharmaceutical combination of compositions and the method of treatment using the combination, respectively, have the advantage of raising an effective antigen-specific T-cell response against cells bearing a target antigen that can be an alloantigen or an autoantigen, especially against a target antigen which is a malignant autoantigen, e.g. raising an antigen-specific T-cell response against cells bearing a tumour-antigen. A further advantage is that the pharmaceutical combination of compositions can raise an antigen-specific T-cell response within a comparatively short time, e.g. within 10 to 14 days following administration of the first composition. The antigen-specific T-cell response is a CD8+ T-cell response, preferably in combination with a CD4+ T-cell response.

### State of art

US 2003/0077263 A1 describes the in vitro production of antigen-presenting dendritic cells for use as a vaccine adjuvant against tumour. CD34+ hematopoietic progenitor cells and stem cells were stimulated with granulocyte-macrophage colony stimulating factor (GM-CSF) for in vitro expansion and differentiation into dendritic cells that were contacted with an antigen or transfected with a gene encoding the antigen, and subsequently activated with a CD40-binding protein. These antigen-pulsed dendritic cells were reintroduced into the original donor of the CD34+ cells.

US 2006/0204509 A1 describes immunization of mice with dendritic cells coated with a peptide representing an epitope of Listerium monocytogenes, followed by a booster immunization with complete Listerium monocytogenes bacteria.

Ahonen et al., J. Exp. Med. 775-784 (2004) describe the immunization of naive mice using concomitant administration of the alloantigen ovalbumin or its epitope peptide SIINFEKL with a TLR agonist and anti-CD40 antibody. The combination of the TLR agonist and anti-CD40 antibody with the antigen was found to induce expansion of antigen-specific CD8+ T-cells. No booster immunization is described.

Poly(I:C) (polyinosinic:polycytidylic acid) is a mismatched double-stranded RNA, one strand being comprised of polyinosinic acid, the other strand of polycytidylic acid. Poly(I:C) is known to interact with TLR3 (Toll-like receptor 3) and is used as an immuno stimulant, e.g. using the sodium salt of Poly(I:C) for simulating viral infections.

### Object of the invention

It is an object of the invention to provide pharmaceutical compositions suitable for effectively raising an immune response, preferably a cellular immune response, especially a CD8+ T-cell response, against cells of a human recipient, which cells bear a target antigen, which preferably is a malignant antigen, e.g. a T-cell response against cancer.

### Description of the invention

The invention achieves the object by the features of the claims, especially by providing a pharmaceutical combination of compounds, which are provided in a first composition of compounds and in a second composition of compounds, for medical use in the treatment or prevention especially of tumours or of infections by virus or intracellular bacteria. The pharmaceutical combination of compounds is provided for administration to a mammal, preferably a human, herein also referred to as a recipient, preferably a human recipient. The first composition is prepared for first administration and the second composition is prepared for separate administration subsequent to administration of the first composition, e.g. for administration subsequent to administration of the first composition by at least 1 to at least 10 days, e.g. subsequent by 2 to 7 days. The pharmaceutical combination of compounds, which are comprised in the first and second compositions, is adapted for eliciting a target antigen-specific CD8+ T-cell response, preferably including a target antigen-specific CD4+ T-cell response, for the prevention and/or treatment of cells bearing the target antigen, which preferably is a malignant antigen, e.g. an autologous tumour antigen for the prevention and/or treatment of tumours bearing tumour antigen. Alternatively, the target antigen is an alloantigen, e.g. an antigen caused by infection by intracellular pathogens, e.g. infections by intracellular bacteria or viral infections and the pharmaceutical combination of compounds is for use in the prevention and/or treatment of infections by a virus or by intracellular bacteria. Accordingly, the pharmaceutical combination is customized for vaccination or for the prevention and/or treatment of tumours or for the prevention and/or treatment of these infections.

Generally, the treatment and the combination of compositions, respectively, can be for neoadjuvant use, e.g. for reduction of the tumour prior to surgery, and/or for adjuvant use, e.g. following tumour surgery, or for palliative use, e.g. without tumour surgery.

The pharmaceutical combination of compounds can be provided for use in the treatment of tumours that can be selected from the group comprising solid cancers and hematological malignancies, e.g. selected from the group comprising or consisting of hematological malignancies, e.g. Hodgkin and non-Hodgkin lymphomas, leukemia, e.g. acute lymphoblastic leukemia, acute myeloid leukemia, chronic lymphocytic leukemia, chronic myeloid leukemia, monocytic leukemia, myelomas, myeloproliferative diseases, myelodysplastic syndromes and solid cancers, e.g. originating from brain, head and neck, lung, pleura, heart, liver, kidney, colon, pancreas, stomach, gut, urinary tract, prostate, uterus, ovaries, breast, skin, testes, larynx and sarcoma.

Further, the invention relates to a method of treatment, raising a target antigen specific immune response, especially a cellular immune response specifically directed against cells bearing a target antigen, which especially is a malignant or a tumour antigen, or a viral antigen or an intracellular bacterial antigen, by administration of the components comprised in the first composition and in the second composition of the pharmaceutical combination to a recipient. Further, the invention relates to the use of the pharmaceutical combination of the first and second compositions in the production of a medicament for the prevention and/or treatment of infection by intracellular pathogens, e.g. infections by intracellular bacteria or viral infections, or for the prevention and/or treatment of tumours, especially of tumours bearing tumour-specific antigen. Accordingly, the invention also relates to a method of prevention and/or treatment of infection by intracellular pathogens, e.g. infections by intracellular bacteria or viral infections, or for the prevention and/or treatment of tumours, especially of tumours bearing tumour-specific antigen.

The first composition of the pharmaceutical combination is customised for priming a target antigen-specific CD8+ T-cell response, preferably inducing the generation of target antigen-specific memory T-cells, and the second composition is customised for boosting the target antigen-specific CD8+ T-cell response. It has been found that the second composition can be customised for administration 2 to 10 days, e.g. 5 to 7 days following administration of the first composition for generating an important antigen-specific CD8+ T-cell response or cell number. Currently it is assumed that target antigen-specific memory T-cells, especially those specific for a target antigen that is a tumour antigen, are induced within at maximum 10, preferably at maximum 7 days following administration of the first composition, and accordingly, the memory T-cells induced by the administration of the combination of compounds of the invention can be described as early memory T-cells. Generally, it is preferred that the administration of the first composition does not induce a systemic inflammation. Accordingly, the target antigen contained in the first composition preferably is an autoantigen, e.g. a tumour antigen, and/or the first composition is free from adjuvants that stimulate an immune response.

Preferably, the immune response additionally induces target antigen-specific CD4+ T-cells that support B-cell mediated antibody production and tumour-specific Th1 T-cell responses. Further, the invention relates to a method for raising an antigen-specific T-cell response in a recipient against cells expressing a target antigen by administration of the pharmaceutical combination, firstly administration of the first composition, and subsequently of the second composition, with a temporal delay of at least 1 day.

The first composition comprises professional antigen presenting cells (APC), which APC preferably express MHC I, preferably dendritic cells (DC), the APC preferably in addition expressing MHC II. Dendritic cells (DCs) are identified by at least one, preferably all of the following surface markers: CD1a, CD1b, CD1c, CD4, CD11c, CD33, CD40, CD80, CD86, CD83, and HLA-DR. DCs include dendritic cell precursor cells, having at least one, preferably all of the following cell surface markers: CD123, CD45RA, CD36, and CD4. The APC are immunologically compatible with the recipient of the pharmaceutical combination, preferably autologous APC, which APC are loaded with the target antigen. The target antigen is an antigen specific for the malignant cells within the recipient, e.g. selected from tumour-specific antigens (malignant antigens), viral antigens and antigens of intracellular bacteria. The APC can be loaded with the target antigen by in vitro contact with the target antigen and/or, for proteinaceous antigen, by in vitro introduction of a nucleic acid sequence encoding the target antigen, e.g. by in vitro transduction or transfection of a nucleic acid sequence encoding the target antigen in an expression cassette.

The APC can originate from the recipient or from an immunologically compatible mammal, preferably a human, e.g. by isolation from peripheral blood. Optionally, the APC can be propagated in vitro by cultivation prior to or following loading with the malignant antigen. For example, APC, e.g. DC can be monocyte-derived DCs or isolated DCs after in vivo induction of DCs. Monocyte-derived DCs can be generated from autologous blood by isolation of monocytes, optional cultivation of monocytes, and differentiation to dendritic cells. In vivo induction of DCs can be obtained by administration of DC growth stimuli, e.g. of flt3 ligand, followed by isolation of DCs, e.g. from peripheral blood.

The first composition can contain the APC which are loaded with a target antigen in a pharmaceutically acceptable formulation that is adapted to keep intact the antigen-loaded APC. Preferably, the first composition is a formulation for intramuscular, sub-cutaneous, intra-venous or intraperitoneal administration. An exemplary formulation of the first composition comprises or consists of immunologically compatible APC loaded with a malignant antigen suspended in physiologic solution, e.g. physiologic saline.

The effective induction of a target antigen-specific immune response by the combination of the first and the second composition, which target antigen preferrably is a tumour antigen, which is autologous, is surprising, because tumours generally evade the immune surveillance and express homologous antigen, against which generally immune responses of only very low magnitude can be elicited. Further, it is surprising that a high number of target antigen-specific T-cells can be induced by administration of the second composition within a short time subsequent to administration of the first composition. The short time delay between administration of the first and of the second compositions allow the use of the combination of the compositions for use in the treatment of tumours or in the treatment of infections by virus or intracellular bacteria, because no long time delay needs to occur before an effective target antigen-specific cellular immune response is induced.

In the alternative to APC loaded with the target antigen, the first composition can comprise the target antigen coupled to an antibody specific for the APC, especially coupled to an antibody specific for a DC surface receptor, e.g. anti-DEC205 antibody or anti-DCIR antibody. In this embodiment, the conjugate comprising the malignant antigen coupled to the antibody specific for the APC following administration of the first composition to the recipient results in the conjugate binding to APC within the recipient and generating APC loaded with the target antigen.

In the embodiments of the invention, the second composition comprises or consists of a target antigen and a co-stimulatory antibody for CD8+-T cells and/or for DC, and preferably a TLR3 agonist, e.g. Poly(I:C) or PolyICLC (polyinosinic-polycytidylic acid stabilized with polylysine and carboxymethylcellulose, available under the trademark Hiltonol), a TLR7 agonist, a TLR4 agonist, a TLR9 agonist and combinations of at least two of these, in a pharmaceutical formulation. The co-stimulatory agonistic antibody for CD8+ T-cells and/or DC is a molecule specifically directed against a surface receptor of T-cells and/or of DCs of the recipient and can e.g. be selected from an anti-CD 137 antibody, an anti-CD40 antibody, an anti-OX40 antibody, an anti-ICOS antibody, an anti-CD27 antibody, an anti-CD28 antibody, an anti-GITR antibody, specifically anti-human GITR/AITR antibody, an anti-HVEM antibody, an anti-TIM1 antibody, an anti-TIM3 antibody, and mixtures of these. The second composition is a formulation for intramuscular, sub-cutaneous, intra-venous or intraperitoneal administration. Optionally, the second composition is provided for systemic administration. The second composition can be provided for administration, e.g. injection, at the same site or at a different site of the recipient's body.

The target antigens of the first composition and of the second composition contain at least one identical epitope for MHC I. Preferably, the malignant antigen of the first composition and the malignant antigen of the second composition share at least one section, the section having an identity of at least 80%, preferably for at least 90%, more preferably for at least 95% or at least 99% of the amino acid sequence. Optionally, the malignant antigens of the first composition and of the second composition are identical.

The malignant antigen preferably is soluble in aqueous media, e.g. in medium containing DCs and/or first composition and in the second composition, and preferably is a proteinaceous antigen, e.g. comprising at least 8 amino acids. Generally, the tumour antigen can be an antigen that is re-expressed in tumour cells or an antigen that is overexpressed in tumour cells, e.g. in comparison to differentiated normal cells. Exemplary tumour antigens of humans are telomerase, oncofetal proteins, e.g. alpha feto protein, and testis antigen, e.g. NY-ESO-1. For example, a tumour specific antigen is one of the group comprising or consisting of tumour antigens resulting from mutations, shared tumour antigens, differentiation antigens, antigens overexpressed in tumours, especially Nras, Hras, Kras which are indicative of a neoplastic state, tumour-specific antigens of the MAGE (including MAGE-B5, MAGE-B6, MAGE, MAGE-C2, MAGE-C3, MAGE-D), HAGE, SAGE, SSX-2, BAGE, TRAG-3, and GAGE families, including NY-ESO-1, LAGE, CAMEL, as well as MUC1, most preferably tumour-specific mutant Ras, e.g. Nras, Nras G12V, or Kras, KrasG12D and other common mutations.

Exemplary tumour antigens resulting from mutations are for lung carcinoma FIASNGVKLV (SEQ ID NO: 1), for melanoma YSVYFNLPADTIYTN(SEQ ID NO: 2), for chronic myeloid leukemia SSKALQRPV (SEQ ID NO: 3) or GFKQSSKAL (SEQ ID NO: 4) or ATGFKQSSKALQRPVAS (SEQ ID NO:5), for melanoma EDLTVKIGDFGLATEKSRWSGSHQFEQLS (SEQ ID NO: 6), for colorectal, gastric, and endometrial carcinoma FLIIWQNTM (SEQ ID NO: 7), for head and neck squamous cell carcinoma FPSDSWCYF (SEQ ID NO: 8), for melanoma SYLDSGIHF (SEQ ID NO: 9), for melanoma FSWAMDLDPKGA (SEQ ID NO: 10), for melanoma ACDPHSGHFV(SEQ ID NO: 11), for melanoma AVCPWTWLR (SEQ ID NO: 12), for colorectal carcinoma TLYQDDTLTLQAAG (SEQ ID NO: 13) or TLYQDDTLTLQAAG (SEQ ID NO: 14), for myeloid leukemia TMKQICKKEIRRLHQY (SEQ ID NO: 15), for melanoma KILDAVVAQK (SEQ ID NO: 16), for lung squamous CC especially ETVSEQSNV (SEQ ID NO: 17), for acute lymphoblastic leukemia RIAECILGM (SEQ ID NO:18) or IGRIAECILGMNPSR (SEQ ID NO:19) or IGRIAECILGMNPSR (SEQ ID NO: 20), for acute myelogenous leukemia YVDFREYEYY (SEQ ID NO: 21), for melanoma MIFEKHGFRRTTPP (SEQ ID NO: 22), for melanoma TLDWLLQTPK (SEQ ID NO: 23), for melanoma WRRAPAPGA (SEQ ID NO: 24) or PVTWRRAPA (SEQ ID NO: 25), for renal cell carcinoma, for melanoma and renal cell carcinoma SLFEGIDIYT (SEQ ID NO: 26), for bladder tumour AEPINIQTW (SEQ ID NO: 27), for melanoma FLEGNEVGKTY (SEQ ID NO: 28), for non-small cell lung carcinoma FLDEFMEGV (SEQ ID NO: 29), for melanoma EEKLIVVLF (SEQ ID NO: 30), for melanoma SELFRSGLDSY (SEQ ID NO: 31) or FRSGLDSYV (SEQ ID NO: 32), for melanoma EAFIQPITR (SEQ ID NO: 33), for melanoma RVIKNSIRLTL (SEQ ID NO: 34), for melanoma KINKNPKYK (SEQ ID NO: 35), lung squamous cell carcinoma QQITKTEV (SEQ ID NO: 36), colorectal carcinoma SLYKFSPFPL (SEQ ID NO: 37), for melanoma KELEGILLL (SEQ ID NO: 38), for head and neck squamous cell carcinoma VVPCEPPEV (SEQ ID NO: 39), for promyelocytic leukemia NSNHVASGAGEAAIETQSSSSEEIV (SEQ ID NO: 40), for melanoma LLLDDLLVSI (SEQ ID NO: 41), for melanoma PYYFAAELPPRNLPEP (SEQ ID NO: 42), for pancreatic adenocarcinoma VVVGAVGVG (SEQ ID NO: 43), for melanoma ILDTAGREEY (SEQ ID NO: 44), for melanoma RPHVPESAF (SEQ ID NO: 45), for melanoma KIFSEVTLK (SEQ ID NO: 46), for melanoma SHETVIIEL (SEQ ID NO: 47), for sarcoma QRPYGYDQIM (SEQ ID NO: 48), for colorectal carcinoma RLSSCVPVA (SEQ ID NO: 49), for melanoma GELIGILNAAKVPAD (SEQ ID NO: 50).

Exemplary shared tumour antigens are:
AARAVFLAL (SEQ ID NO: 51), YRPRPRRY (SEQ ID NO: 52), YYWPRPRRY (SEQ ID NO: 53), VLPDVFIRC(V) (SEQ ID NO: 54), MLAVISCAV (SEQ ID NO: 55), RQKRILVNL (SEQ ID NO: 56), NYNNFYRFL (SEQ ID NO: 57), EYSKECLKEF (SEQ ID NO: 58), EYLSLSDKI (SEQ ID NO: 59), MLMAQEALAFL (SEQ ID NO: 60), SLLMWITQC (SEQ ID NO: 61), LAAQERRVPR (SEQ ID NO: 62), ELVRRILSR (SEQ ID NO: 63), APRGVRMAV (SEQ ID NO: 64), SLLMWITQCFLPVF (SEQ ID NO: 65), QGAMLAAQERRVPRAAEVPR (SEQ ID NO: 66), AADHRQLQLSISSCLQQL (SEQ ID NO:67), CLSRRPWKRSWSAGSCPGMPHL (SEQ ID NO: 68), CLSRRPWKRSWSAGSCPGMPHL (SEQ ID NO: 69), ILSRDAAPLPRPG (SEQ ID NO: 70), AGATGGRGPRGAGA (SEQ ID NO: 71), EADPTGHSY (SEQ ID NO: 72), KVLEYVIKV (SEQ ID NO: 73), SLFRAVITK (SEQ ID NO: 74), EVYDGREHSA (SEQ ID NO: 75), RVRFFFPSL (SEQ ID NO: 76), EADPTGHSY (SEQ ID NO: 77), REPVTKAEML (SEQ ID NO: 78), DPARYEFLW (SEQ ID NO: 79), ITKKVADLVGF (SEQ ID NO: 80), SAFPTTINF (SEQ ID NO: 81), SAYGEPRKL (SEQ ID NO: 82), SAYGEPRKL (SEQ ID NO: 83), TSCILESLFRAVITK (SEQ ID NO: 84), PRALAETSYVKVLEY (SEQ ID NO: 85), FLLLKYRAREPVTKAE (SEQ ID NO: 86), EYVIKVSARVRF (SEQ ID NO: 87), YLQLVFGIEV (SEQ ID NO: 88), EYLQLVFGI (SEQ ID NO: 89), REPVTKAEML (SEQ ID NO: 90), EGDCAPEEK (SEQ ID NO: 91), LLKYRAREPVTKAE (SEQ ID NO: 92), EVDPIGHLY (SEQ ID NO: 93), FLWGPRALV (SEQ ID NO: 94), KVAELVHFL (SEQ ID NO: 95), TFPDLESEF (SEQ ID NO: 96), VAELVHFLL (SEQ ID NO: 97), MEVDPIGHLY (SEQ ID NO: 98), EVDPIGHLY (SEQ ID NO: 99), REPVTKAEML (SEQ ID NO: 100), AELVHFLLL (SEQ ID NO: 101), MEVDPIGHLY (SEQ ID NO: 102), WQYFFPVIF (SEQ ID NO: 103), EGDCAPEEK (SEQ ID NO: 104), KKLLTQHFVQENYLEY (SEQ ID NO: 105), KKLLTQHFVQENYLEY (SEQ ID NO: 106), ACYEFLWGPRALVETS (SEQ ID NO: 107), VIFSKASSSLQL (SEQ ID NO: 108), VIFSKASSSLQL (SEQ ID NO: 109), GDNQIMPKAGLLIIV (SEQ ID NO: 110), TSYVKVLHHMVKISG (SEQ ID NO: 111), RKVAELVHFLLLKYRA (SEQ ID NO: 112), FLLLKYRAREPVTKAE (SEQ ID NO: 113), EVDPASNTY (SEQ ID NO: 114), GVYDGREHTV (SEQ ID NO: 115), NYKRCFPVI (SEQ ID NO: 116), SESLKMIF (SEQ ID NO: 117), MVKISGGPR (SEQ ID NO: 118), EVDPIGHVY (SEQ ID NO: 119), REPVTKAEML (SEQ ID NO: 120), EGDCAPEEK (SEQ ID NO: 121), ISGGPRISY (SEQ ID NO: 122), LLKYRAREPVTKAE (SEQ ID NO: 123), ALSVMGVYV (SEQ ID NO: 124), GLYDGMEHL (SEQ ID NO: 125), DPARYEFLW (SEQ ID NO: 126), FLWGPRALV (SEQ ID NO: 127), VRIGHLYIL (SEQ ID NO: 128), EGDCAPEEK (SEQ ID NO: 129), REPFTKAEMLGSVIR (SEQ ID NO: 130), AELVHFLLLKYRAR (SEQ ID NO: 131), LLFGLALIEV (SEQ ID NO: 132), ALKDVEERV (SEQ ID NO: 133), SESIKKKVL (SEQ ID NO: 134), PDTRPAPGSTAPPAHGVTSA (SEQ ID NO: 135), QGQHFLQKV (SEQ ID NO: 136), SLLMWITQC (SEQ ID NO: 137), MLMAQEALAFL (SEQ ID NO: 138), ASGPGGGAPR (SEQ ID NO: 139), LAAQERRVPR (SEQ ID NO: 140), TVSGNILTIR (SEQ ID NO: 141), APRGPHGGAASGL (SEQ ID NO: 142), MPFATPMEA (SEQ ID NO: 143), KEFTVSGNILTI (SEQ ID NO: 144), MPFATPMEA (SEQ ID NO: 145), LAMPFATPM (SEQ ID NO: 146), ARGPESRLL (SEQ ID NO: 147), SLLMWITQCFLPVF (SEQ ID NO: 148), LLEFYLAMPFATPMEAELARRSLAQ (SEQ ID NO: 149), LLEFYLAMPFATPMEAELARRSLAQ (SEQ ID NO: 150), EFYLAMPFATPM (SEQ ID NO: 151), RLLEFYLAMPFA (SEQ ID NO: 152), QGAMLAAQERRVPRAAEVPR (SEQ ID NO: 153), PGVLLKEFTVSGNILTIRLT (SEQ ID NO: 154), VLLKEFTVSG (SEQ ID NO: 155), AADHRQLQLSISSCLQQL (SEQ ID NO: 156), LLEFYLAMPFATPMEAELARRSLAQ (SEQ ID NO: 157), LKEFTVSGNILTIRL (SEQ ID NO:158 ), PGVLLKEFTVSGNILTIRLTAADHR (SEQ ID NO: 159), LLEFYLAMPFATPMEAELARRSLAQ (SEQ ID NO: 160), AGATGGRGPRGAGA (SEQ ID NO: 161), LYATVIHDI (SEQ ID NO: 162), ILDSSEEDK (SEQ ID NO: 163), KASEKIFYV (SEQ ID NO: 164), EKIQKAFDDIAKYFSK (SEQ ID NO: 165), WEKMKASEKIFYVYMKRK (SEQ ID NO: 166), KIFYVYMKRKYEAMT (SEQ ID NO: 167), KIFYVYMKRKYEAM (SEQ ID NO: 168), INKTSGPKRGKHAWTHRLRE (SEQ ID NO: 169), YFSKKEWEKMKSSEKIVYVY (SEQ ID NO: 170), MKLNYEVMTKLGFKVTLPPF (SEQ ID NO: 171), KHAWTHRLRERKQLVVYEEI (SEQ ID NO: 172), LGFKVTLPPFMRSKRAADFH (SEQ ID NO: 173), KSSEKIVYVYMKLNYEVMTK (SEQ ID NO: 174), KHAWTHRLRERKQLVVYEEI (SEQ ID NO: 175), SLGWLFLLL (SEQ ID NO: 176), LSRLSNRLL (SEQ ID NO: 177), LSRLSNRLL (SEQ ID NO: 178), CEFHACWPAFTVLGE (SEQ ID NO: 179), CEFHACWPAFTVLGE (SEQ ID NO: 180), CEFHACWPAFTVLGE (SEQ ID NO: 181), EVISCKLIKR (SEQ ID NO: 182) or CATWKVICKSCISQTPG (SEQ ID NO: 183).

Exemplary tumour differentiation antigens are:
YLSGANLNL (SEQ ID NO: 184), IMIGVLVGV (SEQ ID NO: 185), GVLVGVALI (SEQ ID NO: 186), HLFGYSWYK (SEQ ID NO: 187), QYSWFVNGTF (SEQ ID NO: 188), TYACFVSNL (SEQ ID NO: 189), AYVCGIQNSVSANRS (SEQ ID NO: 190), DTGFYTLHVIKSDLVNEEATGQFRV (SEQ ID NO: 191), YSWRINGIPQQHTQV (SEQ ID NO: 192), TYYRPGVNLSLSC (SEQ ID NO: 193), EIIYPNASLLIQN (SEQ ID NO: 194), YACFVSNLATGRNNS (SEQ ID NO: 195), LWWVNNQSLPVSP (SEQ ID NO: 196), LWWVNNQSLPVSP (SEQ ID NO:197), LWWVNNQSLPVSP (SEQ ID NO:198), EIIYPNASLLIQN (SEQ ID NO: 199), NSIVKSITVSASG (SEQ ID NO: 200), KTWGQYWQV (SEQ ID NO: 201), (A)MLGTHTMEV (SEQ ID NO: 202), ITDQVPFSV (SEQ ID NO: 203), YLEPGPVTA (SEQ ID NO: 204), LLDGTATLRL (SEQ ID NO: 205), VLYRYGSFSV (SEQ ID NO: 206), SLADTNSLAV (SEQ ID NO: 207), RLMKQDFSV (SEQ ID NO: 208), RLPRIFCSC (SEQ ID NO: 209), LIYRRRLMK (SEQ ID NO: 210), ALLAVGATK (SEQ ID NO: 211), IALNFPGSQK (SEQ ID NO: 212), ALNFPGSQK (SEQ ID NO: 213), ALNFPGSQK (SEQ ID NO: 214), VYFFLPDHL (SEQ ID NO: 215), RTKQLYPEW (SEQ ID NO: 216), HTMEVTVYHR (SEQ ID NO: 217), SSPGCQPPA (SEQ ID NO: 218), VPLDCVLYRY (SEQ ID NO: 219), LPHSSSHWL (SEQ ID NO: 220), SNDGPTLI (SEQ ID NO: 221), GRAMLGTHTMEVTVY (SEQ ID NO: 222), WNRQLYPEWTEAQRLD (SEQ ID NO: 223), TTEWVETTARELPIPEPE (SEQ ID NO: 224), TGRAMLGTHTMEVTVYH (SEQ ID NO: 225), GRAMLGTHTMEVTVY (SEQ ID NO: 226), SVSESDTIRSISIAS (SEQ ID NO: 227), LLANGRMPTVLQCVN (SEQ ID NO: 228), RMPTVLQCVNVSVVS (SEQ ID NO: 229), PLLENVISK (SEQ ID NO: 230), (E)AAGIGILTV (SEQ ID NO: 231), ILTVILGVL (SEQ ID NO: 232), EAAGIGILTV (SEQ ID NO: 234), AEEAAGIGIL(T) (SEQ ID NO: 235), RNGYRALMDKS (SEQ ID NO: 236), EEAAGIGILTVI (SEQ ID NO: 237), AAGIGILTVILGVL (SEQ ID NO: 238), APPAYEKLpSAEQ (SEQ ID NO: 239), EEAAGIGILTVI (SEQ ID NO: 240), RNGYRALMDKSLHVGTQCALTRR (SEQ ID NO: 241), MPREDAHFIYGYPKKGHGHS (SEQ ID NO: 242), KNCEPVVPNAPPAYEKLSAE (SEQ ID NO: 243), SLSKILDTV (SEQ ID NO: 244), LYSACFWWL (SEQ ID NO: 245), FLTPKKLQCV (SEQ ID NO: 246), VISNDVCAQV (SEQ ID NO: 247), VLHWDPETV (SEQ ID NO: 248), MSLQRQFLR (SEQ ID NO: 249), ISPNSVFSQWRVVCDSLEDYD (SEQ ID NO: 250), SLPYWNFATG (SEQ ID NO: 251), SVYDFFVWL (SEQ ID NO: 252), TLDSQVMSL (SEQ ID NO: 253), LLGPGRPYR (SEQ ID NO: 254), LLGPGRPYR (SEQ ID NO: 255), ANDPIFVVL (SEQ ID NO: 256), QCTEVRADTRPWSGP (SEQ ID NO: 257), ALPYWNFATG (SEQ ID NO: 258), KCDICTDEY (SEQ ID NO: 259), SSDYVIPIGTY (SEQ ID NO: 260), MLLAVLYCL (SEQ ID NO: 261), CLLWSFQTSA (SEQ ID NO: 262), YMDGTMSQV (SEQ ID NO: 263), AFLPWHRLF (SEQ ID NO: 264), QCSGNFMGF (SEQ ID NO: 265), TPRLPSSADVEF (SEQ ID NO: 266), LPSSADVEF (SEQ ID NO: 267), LHHAFVDSIF (SEQ ID NO: 268), SEIWRDIDF (SEQ ID NO: 269), QNILLSNAPLGPQFP (SEQ ID NO: 270), SYLQDSDPDSFQD (SEQ ID NO: 271) or FLLHHAFVDSIFEQWLQRHRP (SEQ ID NO: 272).

Exemplary antigens overexpressed in tumour are:
SVASTITGV (SEQ ID NO: 273), RSDSGQQARY (SEQ ID NO: 274), LLYKLADLI (SEQ ID NO: 275), YLNDHLEPWI (SEQ ID NO: 276), CQWGRLWQL (SEQ ID NO: 277), VLLQAGSLHA (SEQ ID NO: 278), KVHPVIWSL (SEQ ID NO: 279), LMLQNALTTM (SEQ ID NO: 280), LLGATCMFV (SEQ ID NO: 281), NPPSMVAAGSVVAAV (SEQ ID NO: 282), ALGGHPLLGV (SEQ ID NO: 283), TMNGSKSPV (SEQ ID NO: 284), RYQLDPKFI (SEQ ID NO: 285), DVTFNIICKKCG (SEQ ID NO: 286), FMVEDETVL (SEQ ID NO: 287), FINDEIFVEL (SEQ ID NO: 288), KYDCFLHPF (SEQ ID NO: 289), KYVGIEREM (SEQ ID NO: 290), NTYASPRFK (SEQ ID NO: 291), HLSTAFARV (SEQ ID NO: 292), KIFGSLAFL (SEQ ID NO: 293), IISAWGIL (SEQ ID NO: 294), ALCRWGLLL (SEQ ID NO: 295), ILHNGAYSL (SEQ ID NO: 296), RLLQETELV (SEQ ID NO: 297), VVLGVVFGI (SEQ ID NO: 298), YMIMVKCWMI (SEQ ID NO: 299), HLYQGCQVV (SEQ ID NO: 300), YLVPQQGFFC(SEQ ID NO: 301), PLQPEQLQV (SEQ ID NO: 302), TLEEITGYL (SEQ ID NO: 303), ALIHHNTHL (SEQ ID NO: 304), PLTSIISAV (SEQ ID NO: 305), VLRENTSPK (SEQ ID NO: 306), TYLPTNASL (SEQ ID NO: 307), ALLEIASCL (SEQ ID NO: 308), WLPFGFILI (SEQ ID NO: 309), SPRWWPTCL (SEQ ID NO: 310), GVALQTMKQ (SEQ ID NO: 311), FMNKFIYEI (SEQ ID NO: 312), QLAVSVILRV (SEQ ID NO: 313), LPAVVGLSPGEQEY (SEQ ID NO: 314), VGQDVSVLFRVTGALQ (SEQ ID NO: 315), VLFYLGQY (SEQ ID NO: 316), TLNDECWPA (SEQ ID NO: 317), GLPPDVQRV (SEQ ID NO: 318), SLFPNSPKWTSK (SEQ ID NO: 319), STAPPVHNV (SEQ ID NO: 320), LLLLTVLTV (SEQ ID NO: 321), PGSTAPPAHGVT (SEQ ID NO: 322), LLGRNSFEV (SEQ ID NO: 323), RMPEAAPPV (SEQ ID NO: 324), SQKTYQGSY (SEQ ID NO: 325), PGTRVRAMAIYKQ (SEQ ID NO: 326), HLIRVEGNLRVE (SEQ ID NO: 327), TLPGYPPHV (SEQ ID NO: 328), CTACRWKKACQR (SEQ ID NO: 329), VLDGLDVLL (SEQ ID NO: 330), SLYSFPEPEA (SEQ ID NO: 331), ALYVDSLFFL (SEQ ID NO: 332), SLLQHLIGL (SEQ ID NO: 333), LYVDSLFFL (SEQ ID NO: 334), NYARTEDFF (SEQ ID NO: 335), LKLSGVVRL (SEQ ID NO: 336), PLPPARNGGL (SEQ ID NO: 337), SPSSNRIRNT (SEQ ID NO: 338), LAALPHSCL (SEQ ID NO: 339), GLASFKSFLK(SEQ ID NO: 340), RAGLQVRKNK (SEQ ID NO: 341), ALWPWLLMA(T) (SEQ ID NO: 342), NSQPVWLCL (SEQ ID NO: 343), LPRWPPPQL (SEQ ID NO: 344), KMDAEHPEL (SEQ ID NO: 345), AWISKPPGV (SEQ ID NO: 346), SAWISKPPGV (SEQ ID NO: 347), MIAVFLPIV (SEQ ID NO: 348), HQQYFYKIPILVINK (SEQ ID NO: 349), ELTLGEFLKL (SEQ ID NO: 350), ILAKFLHWL (SEQ ID NO: 351), RLVDDFLLV (SEQ ID NO: 352), RPGLLGASVLGLDDI (SEQ ID NO: 353), LTDLQPYMRQFVAHL (SEQ ID NO: 354), SRFGGAVVR (SEQ ID NO: 355), TSEKRPFMCAY (SEQ ID NO: 356), CMTWNQMNL (SEQ ID NO: 357), LSHLQMHSRKH (SEQ ID NO: 358) or KRYFKLSHLQMHSRKH (SEQ ID NO: 359)

A preferred tumour antigen is a lysate or homogenate of the tumour to be treated, more preferably a fraction thereof soluble in aqueous media, e.g. soluble in physiological saline and/or in medium containing DCs. Tumour lysate or tumour homogenate can e.g. originate from a tumour biopsy or from a surgery of the later recipient of the pharmaceutical combination of compounds.

It has been found that the administration of the first composition and subsequent administration of the second composition results in the generation of target antigen-specific activated T-cells. In comparison to other compositions, the combination of the invention results also in a higher proportion of target antigen-specific activated T-cells of all activated T-cells. The number and proportion of target antigen-specific T-cells was determined by staining a peripheral blood sample for IFN gamma following in vitro contacting with the target antigen using brefeldin A (GolgiPlug, available from Becton Dickinson) and immuno staining with a labelled anti-IFN gamma antibody and detection in flow cytometry. The number of all activated T-cells was determined by staining a peripheral blood sample with anti-CD11a antibody and detection in flow cytometry. The effect of inducing a target antigen-specific T-cell response, wherein the target antigen preferably is a tumour antigen, within e.g. 10 to 14 days is currently believed to be based on the combination of the first composition providing APC specifically primed for the target antigen, which APC preferably are DC, with the second composition providing a specific boost for the T-cells that were stimulated by the DCs loaded with target antigen by the combination of the malignant antigen with the co-stimulatory antibody for T-cells, preferably in combination with the non-specific agonist for TLR3, for TLR7, for TLR4 and/or for TLR9. Accordingly, the first composition and its administration can also be termed priming, and the second composition and its administration can be termed boosting.

In the embodiment in which the APC loaded with target antigen contained in the first composition are replaced with target antigen coupled to an antibody specific for the APC, especially coupled to an antibody specific for a DC surface receptor, e.g. anti-DEC205 antibody or anti-DCIR antibody, the target antigen-loaded APC are generated vivo by administration of the first composition.

It is currently assumed that the second composition due to its content of the target antigen preferentially boosts malignant antigen-specific T-cells from the pool of primed T-cells present in the recipient. This is an advantage over the use of co-stimulating antibody and/or of a non-specific TLR3 agonist alone or in combination as these can be expected to activate all primed T-cells irrespective of their antigen specificity, resulting in a proportionate boost of target antigen-specific T-cells only. Accordingly, the second composition is also assumed to have the advantage of boosting non-target specific T-cells to a lesser extent than the use of co-stimulating antibody and/or of a non-specific TLR3 agonist alone.

Advantageously, the administration of the second composition following the administration of the first composition is with a temporal delay of approx. 1 to 7 days.

The pharmaceutical combination of compounds has the advantage of raising in a recipient an effective T-cell immunity also against an intracellular tumour antigen.

For the purpose of the invention, an antibody, e.g. an antibody specific for a DC surface receptor, a co-stimulatory agonistic antibody for CD8+ T-cells, can be a natural antibody, e.g. IgG, or a synthetic peptide having a paratope of the specificity, e.g. a diabody, minibody etc. The invention is now described in greater detail by way of mouse experiments with reference to the figures, which show for different first and second compositions administered to experimental animals in
- Fig. 1 flow cytometry results of peripheral blood with staining for CD11a at day -1 prior to administration of the second composition at a), c), e), g), and i) with restimulation with the target antigen (Ndufs1) and at b), d), f), h) and j) without restimulation (Control) with antigen,
- Fig. 2 flow cytometry results of peripheral blood with staining for IFN gamma at day - 1 prior to administration of the second composition at a), c), e), g), and i) with restimulation with the target antigen (Ndufs1) and at b), d), f), h) and j) without restimulation (Control) with antigen,
- Fig. 3 a graphical representation of the proportion of malignant antigen-specific CD8+ T-cells from the results of Fig. 2,
- Fig. 4 a) - j) flow cytometry results of peripheral blood with staining for highly CD110a-positive T-cells at day 7 following administration of the second composition,
- Fig. 5 a graphical representation of the proportion of activated, i.e. highly CD11a-positive T-cells from the results of Fig. 4,
- Fig. 6 flow cytometry results of peripheral blood with staining for IFN gamma-positive T-cells at day 7 following administration of the second composition at a), c), e), g), and i) with restimulation with the target antigen (Ndufs1) and at b), d), f), h) and j) without restimulation (Control) with antigen, and

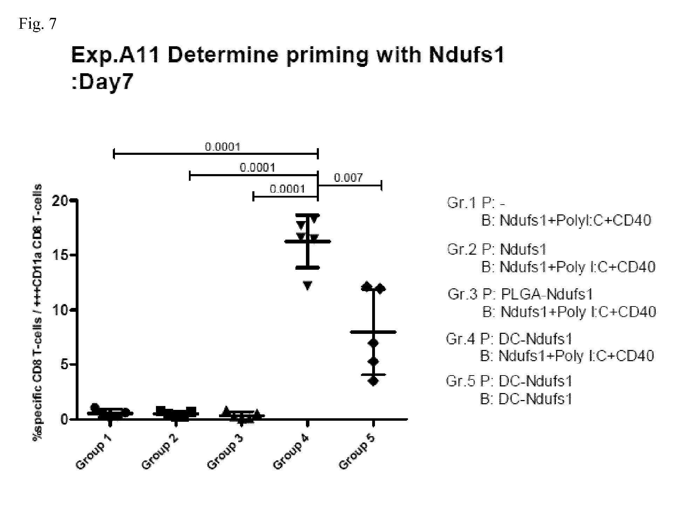

- Fig. 7 a graphical representation of the proportion of IFN gamma-positive T-cells in activated T-cells from the results of Fig. 6.

In the following examples and comparative examples, mice were used for representing a human recipient. Mice were divided into groups of 5 mice (strain C57 B1/6) each. The animals were housed under standard conditions with feed and water ad libitum. Mice were subjected to different prime-boost regimens. Administration of first composition (priming) and of second composition (boosting) was by intravenous (iv) injection.

As an example for a malignant antigen, a mouse antigen isolated from HCC tumour, Ndufs1 having amino acid AAVSNMVQKI (SEQ ID NO: 360) was used. Ndufs1 was prepared by chemical peptide synthesis. The compositions comprised the constituents of the compositions in aqueous medium, preferably in physiological saline.

### Example 1: Immunization with different first compositions, followed by different second compositions

For priming, on day -7 mice received as a first composition either physiological saline (group 1), 100µg soluble Ndufs1 peptide (group 2), 100µg Ndufs peptide conjugated to 1mg PLGA microspheres of 2µm mean diameter (group 3), or 10⁶ dendritic cells that were in vitro coated with 10µg Ndufs1 peptide (groups 4 and 5) intravenously. 7 days later (day 0), mice received boosting by intravenous administration of a combination of 100µg Ndufs peptide, 100µg of agonistic anti-CD40 antibody (clone 1C10) and 200µg of Poly(I:C) (groups 1 to 4), or again 10⁶ dendritic cells that were in vitro coated with 10µg Ndufs1 peptide (group 5) as the second composition. After the administration of the second composition, mice were bled from the mandibular vein on the days indicated below. After red cell lysis, peripheral blood mononuclear cells were stained with the following labelled antibodies: anti-IFN gamma antibody-APC (clone XMG1.2, eBioscience), anti-CD8 antibody-FITC (53-6.7, eBioscience and Becton Dickinson Biosciences), anti-CD11a antibody-PE (M17/4, eBioscience).

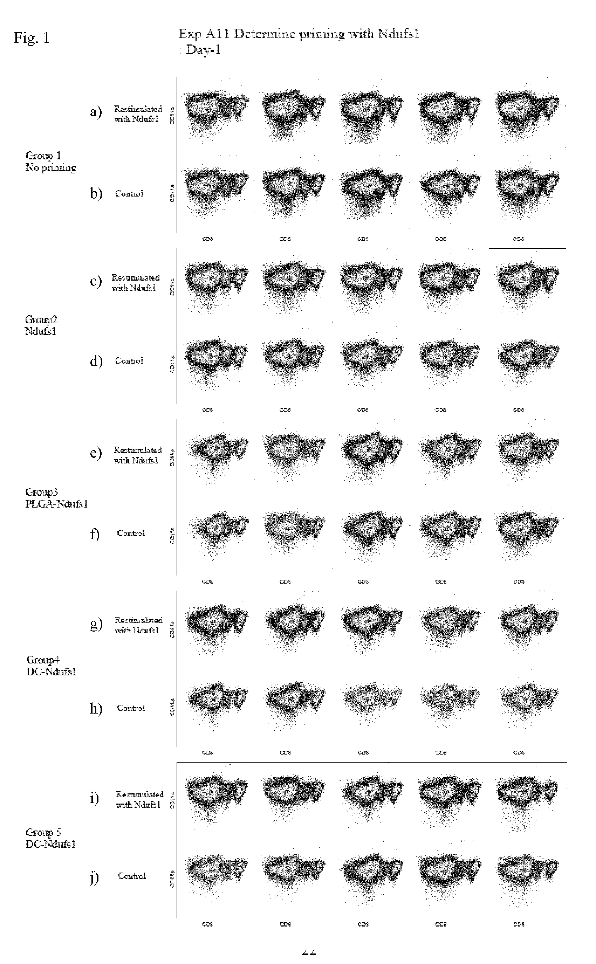

The results of flow cytometry using a model Canto II flow cytometer (Becton Dickinson Biosciences) are shown in Fig. 1.

The following table summarizes first compositions followed by administration of the second compositions:

| Group (Gr.) | results in | priming | boosting |
|---|---|---|---|
| 1 | Fig. 1a), 1b) | physiological saline (No priming) | Ndufs1 + Poly (I:C) + anti-CD40 |
| | Fig. 2a), 2b) | | |
| 2 | Fig. 1c), d) | Ndufs1 only (Ndufs1) | Ndufs1 + Poly (I:C) + anti-CD40 |
| | Fig. 2c), d) | | |
| 3 | Fig. 1e), f) | PLGA-Ndufs1 | Ndufs1+ Poly (I:C) + anti-CD40 |
| | Fig. 2e), f) | | |
| 4 | Fig. 1g), h) | DC-Ndufs1 | Ndufs1+Poly (I:C) + anti-CD40 |
| | Fig. 2g), h) | | |
| 5 | Fig. 1i), j) | DC-Ndufs1 | DC-Ndufs1 |
| | Fig. 2i), j) | | |

The 5 animals of each group were treated identically.

PLGA-Ndufs1 designates microspheres of poly(lactic-co-glycolic) acid comprising the model antigen Ndufs1. DC-Ndufs1 designates dendritic cells (DCs) isolated from the spleen of a mouse of the same strain without administration of the antigen Ndufs1, which DCs were incubated in RPMI culture medium and loaded with the antigen by adding Ndufs to a concentration of approx. 2 µg/ml medium.

Figures 1-3 refer to analyses at day -1, i.e. 6 days following administration of the respective first composition and 1 day prior to administration of the respective second composition.

Figure 1 shows the results of analysis for CD11a, indicating the activated T-cells of the total T-cells (CD8), with addition of antigen Ndufs1 (Figs. a), c), e), g) and i)) in one sample of each animal and without added antigen (Control) for each sample of each animal (Figs. b), d), f), h) and j)).

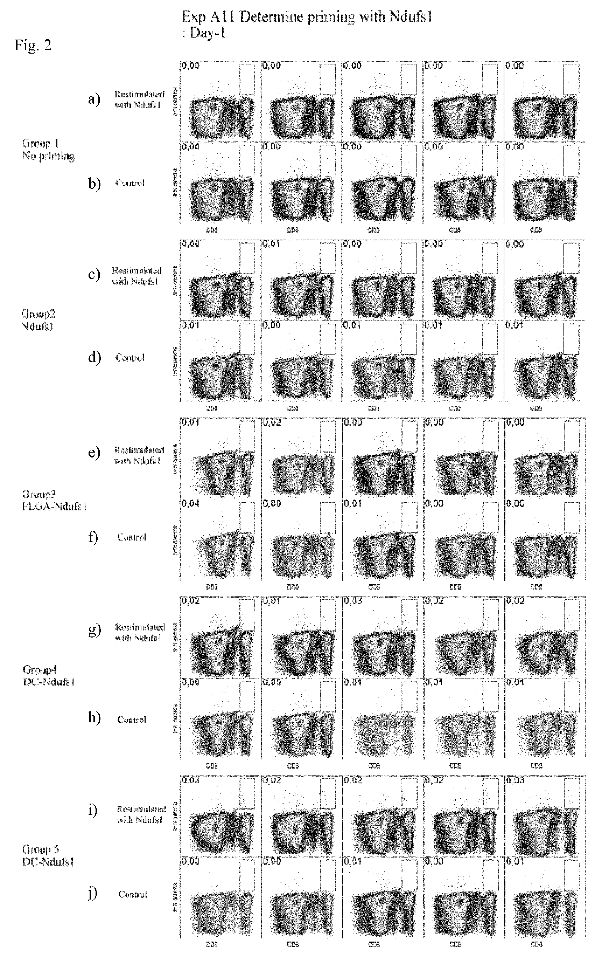

The analysis for target antigen-specific T-cells was by measuring IFN gamma following restimulation with antigen Ndufs1 (Figs. 2 a), c), e), g) and i)) in a sample of each animal in comparison to the samples from the same animals without added antigen (Figs. 2 b), d), f), h) and j)). For measurement of IFN gamma produced by each T-cell (CD8), secretion of IFN gamma was hindered by addition of brefeldin A (GolgiPlug, available from Becton Dickinson), followed by staining using a labelled anti-IFN gamma antibody and measurement by flow cytometry.

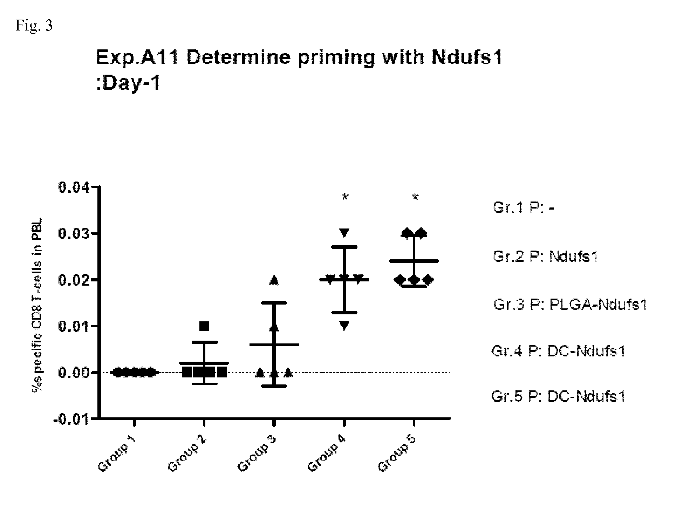

The flow cytometry analyses after administration of the first compositions only (P, priming) are summarized in Fig. 3, showing that a first composition consisting of DCs in vitro loaded the model tumour antigen Ndufs in groups 4 and 5 (DC-Ndufs1) resulted in approx. 0.02 % specific CD8+ T-cells in peripheral blood lymphocytes (PBL), whereas priming with PLGA microspheres with the antigen, group 3 (PLGA-Ndufs1) or antigen alone in group 2 (Ndufs1) resulted in numbers of antigen-specific CD8+ T-cells in PBL close to background obtained without antigen (Gr. 1, -).

Following administration of the second compositions at day 0 to the same animals, samples were taken 7 days later (Day 7).

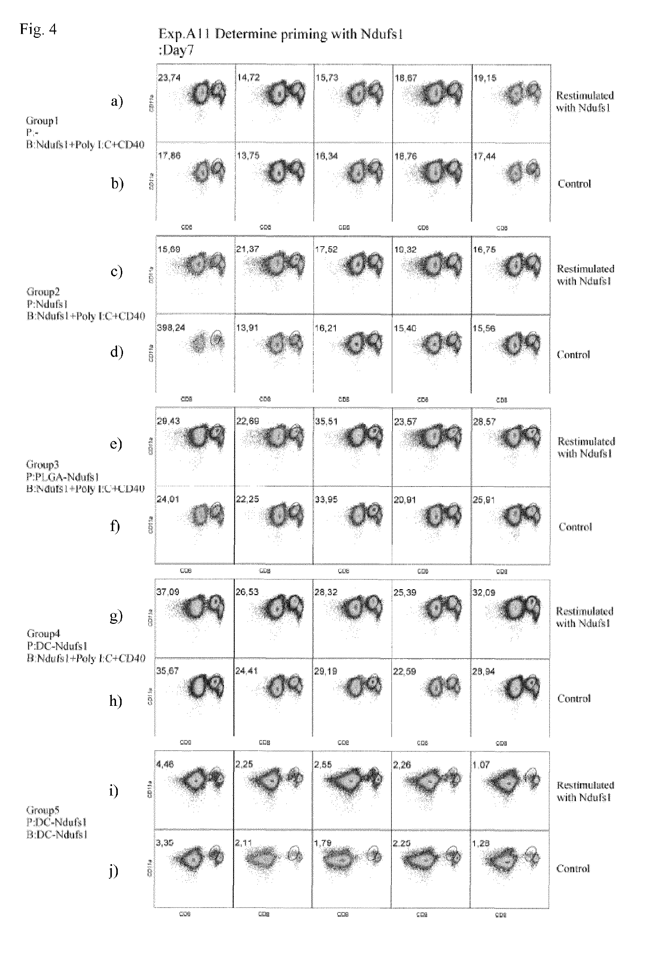

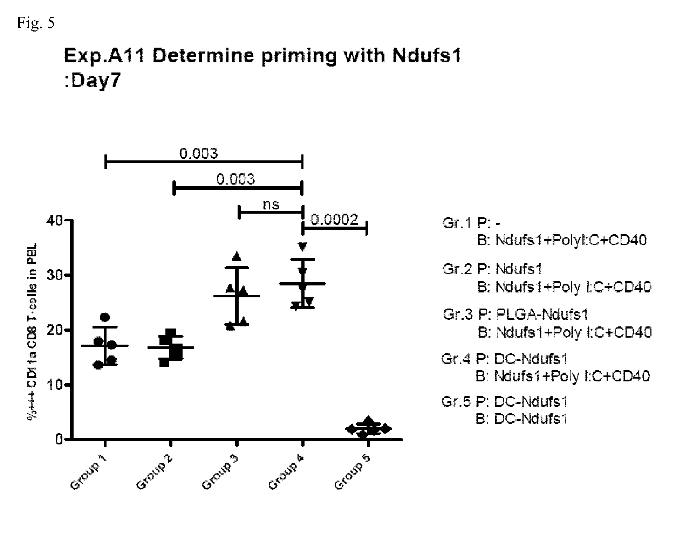

The flow cytometry results from Fig. 4 for staining with anti-CD 11a antibody (CD 11a) and anti-CD8 antibody (CD8), using the encircled areas, are summarized in Fig. 5, showing that CD 11a high positive T-cells (+++CD11a), which are activated T-cells, are generated to approx. 27-28 % by the first compositions ofPLGA microspheres comprising the antigen or DC loaded with antigen, followed by administration of the second composition comprising the antigen, the TLR3 agonist Poly(I:C) and the co-stimulating antibody anti-CD40 antibody. In contrast, the priming with antigen-primed DC followed by boosting with antigen-primed DC gave the lowest number of activated CD8+ T-cells.

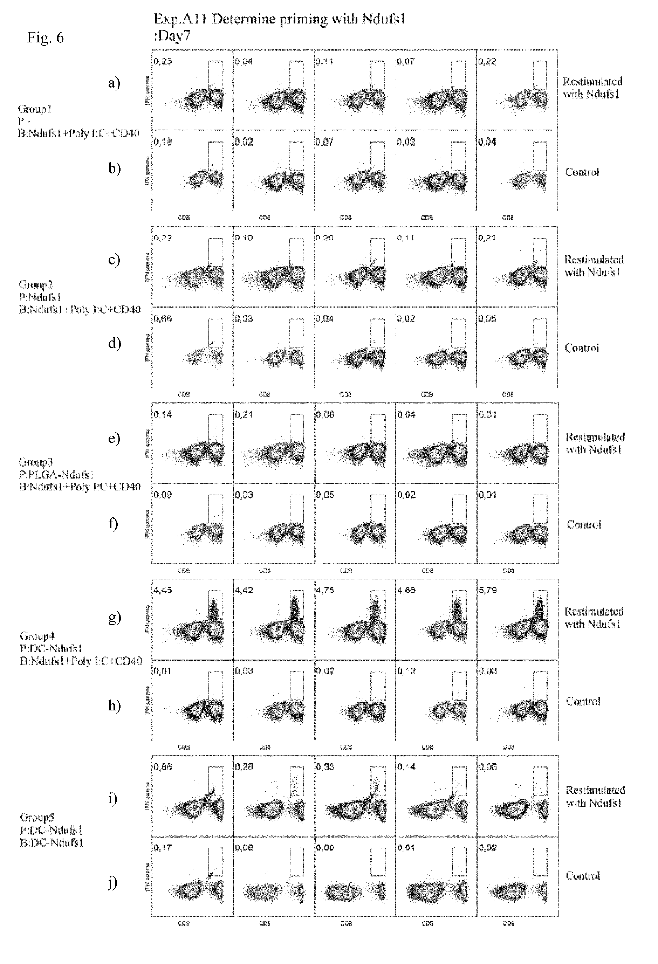

Fig. 6 shows the flow cytometry results for staining with anti-IFN gamma (IFN gamma) and anti-CD8+ (CD8), following restimulation with antigen Ndufs1 (Figs. 6 a), c), e), g) and i)) and without added antigen (Figs. 6 b), d), f), h) and j)). It is clearly seen that the priming with a first composition comprising DC loaded with antigen followed by boosting with a second composition comprising the antigen in combination with the TLR3 agonist and with the co-stimulating antibody according to the invention yields the most effective generation of a proportion of antigen-specific CD8+ T-cells (insets in Fig. 6g)) in activated T-cells, which are highly CD11a positive CD8+ T-cells (+++CD11a CD8 T-cells), approx. 16% (Group 4). This proportion is significantly higher than that obtained for both priming and boosting by antigen-loaded DC (Group 5).

## Claims

1. Pharmaceutical combination of compositions for use in medical treatment, the combination comprising a first composition comprising professional antigen presenting cells (APC), preferably dendritic cells (DCs), which professional antigen presenting cells (APC) are immunologically compatible with a recipient and which are associated with a target antigen and a second composition comprising at least a portion of the target antigen in soluble form and a co-stimulatory antibody effective for activating T-cells and/or antigen presenting cells (APC), wherein the second composition is for administration at a time at least 1 day subsequent to administration of the first composition.

2. Pharmaceutical combination according to claim 1, wherein the professional antigen presenting cells (APC) are associated with the target antigen by being contacted with the target antigen or by being contacted with a nucleic acid sequence encoding the antigen.

3. Pharmaceutical combination of compositions for use in medical treatment, the combination comprising a first composition comprising an antibody specific for a surface receptor of a dendritic cell (DC) coupled to a target antigen and a second composition comprising at least a portion of the target antigen in soluble form and a co-stimulatory antibody effective for activating T-cells and/or antigen presenting cells (APC), wherein the second composition is provided for administration at a time at least 1 day subsequent to administration of the first composition.

4. Pharmaceutical combination according to claim 3, wherein the antibody specific for a surface receptor of a dendritic cell (DC) is an anti-DEC205 antibody and/or an anti-DCIR antibody.

5. Pharmaceutical combination according to one of the preceding claims, wherein the medical treatment is the treatment of tumour, of viral infections or of infections by intracellular bacteria.

6. Pharmaceutical combination according to one of the preceding claims, wherein the second composition further contains a non-specific TLR3 agonist, TLR7 agonist, TLR4 agonist, TLR9 agonist or combinations of at least two of these.

7. Pharmaceutical combination according to one of the preceding claims, wherein the co-stimulatory antibody effective for activating professional antigen presenting cells (APC) is selected from the group consisting of anti-CD 137 antibody, an anti-CD40 antibody, an anti-OX40 antibody, anti-ICOS antibody, an anti-CD27 antibody, an anti-CD28 antibody, an anti-GITR antibody, specifically anti-human GITR/AITR antibody, an anti-HVEM antibody, an anti-TIM1 antibody, an anti-TIM3 antibody, and mixtures of at least two of these.

8. Pharmaceutical combination according to one of claims 3 to 4, wherein the non-specific TLR3 agonist is Poly(I:C) and/or PolyICLC or a homologue thereof.

9. Pharmaceutical combination according to one of the preceding claims, wherein the medical treatment is for raising in a recipient a cellular immune response specifically directed against cells of the recipient bearing the target antigen.

10. Pharmaceutical combination according to one of the preceding claims, wherein the first composition is free from an adjuvant.

11. Pharmaceutical combination according to one of claims 5 to 10, wherein the tumour is selected from the group comprising or consisting of hematological malignancies, Hodgkin and non-Hodgkin lymphomas, leukemias, especially acute lymphoblastic leukemia, acute myeloid leukemia, chronic lymphocytic leukemia, chronic myeloid leukemia, monocytic leukemia, myelomas, myeloproliferative diseases, myelodysplastic syndromes and solid cancers, especially originating from brain, head and neck, lung, pleura, heart, liver, kidney, colon, pancreas, stomach, gut, urinary tract, prostate, uterus, ovaries, breast, skin, testes, larynx and sarcoma.

12. Pharmaceutical combination according to one of the preceding claims, wherein the tumour antigen is selected from the group consisting of tumour homogenate or tumour lysate.

13. Pharmaceutical combination according to one of claim 2 or claims 5 to 12, wherein the professional antigen presenting cells (APC) following in vitro contact with the target antigen by being contacted with the target antigen or by being contacted with a nucleic acid sequence encoding the antigen are separated from the medium containing the target antigen or nucleic acid sequence encoding the antigen and are expanded in number by cultivation in cell culture medium.

14. Pharmaceutical combination according to one of the preceding claims, wherein the medical treatment comprises the generation of CD8+ T-cells which are specific for the target antigen and/or the generation of CD4+ T-cells which are specific for the target antigen.


SEQUENCE LISTING

<110> Medizinische Hochschule Hannover

<120> Vaccine

<130> M1056EP-N

<150> EP14166480
<151> 2014-04-29

<160> 360

<170> BiSSAP 1.2

<210> 1
<211> 10
<212> PRT
<213> Artificial Sequence

<220>
<223> tumour antigen for lung carcinoma

<400> 1
Phe Ile Ala Ser Asn Gly Val Lys Leu Val
1               5                   10

<210> 2
<211> 15
<212> PRT
<213> Artificial Sequence

<220>
<223> tumour antigen for melanoma

<400> 2
Tyr Ser Val Tyr Phe Asn Leu Pro Ala Asp Thr Ile Tyr Thr Asn
1               5                   10                  15

<210> 3
<211> 9
<212> PRT
<213> Artificial Sequence

<220>
<223> tumour antigen for chronic myeloid leukemia

<400> 3
Ser Ser Lys Ala Leu Gln Arg Pro Val
1               5

<210> 4
<211> 9
<212> PRT
<213> Artificial Sequence

<220>
<223> tumour antigen for chronic myeloid leukemia

<400> 4
Gly Phe Lys Gln Ser Ser Lys Ala Leu
1               5

<210> 5
<211> 17
<212> PRT
<213> Artificial Sequence

<220>
<223> tumour antigen for chronic myeloid leukemia

<400> 5
Ala Thr Gly Phe Lys Gln Ser Ser Lys Ala Leu Gln Arg Pro Val Ala
1               5                   10                  15
Ser


<210> 6
<211> 17
<212> PRT
<213> Artificial Sequence

<220>
<223> tumour antigen for chronic myeloid leukemia

<400> 6
Ala Thr Gly Phe Lys Gln Ser Ser Lys Ala Leu Gln Arg Pro Val Ala
1               5                   10                  15
Ser


<210> 7
<211> 29
<212> PRT
<213> Artificial Sequence

<220>
<223> tumour antigen for melanoma

<400> 7
Glu Asp Leu Thr Val Lys Ile Gly Asp Phe Gly Leu Ala Thr Glu Lys
1               5                   10                  15
Ser Arg Trp Ser Gly Ser His Gln Phe Glu Gln Leu Ser
            20                  25

<210> 8
<211> 9
<212> PRT
<213> Artificial Sequence

<220>
<223> tumour antigen for colorectal, gastgric , and endometrialt umour

<400> 8
Phe Leu Ile Ile Trp Gln Asn Thr Met
1               5

<210> 9
<211> 9
<212> PRT
<213> Artificial Sequence

<220>
<223> tumour antigen for head and neck squamous cell carcinoma

<400> 9

Phe Pro Ser Asp Ser Trp Cys Tyr Phe
1               5

<210> 10
<211> 9
<212> PRT
<213> Artificial Sequence

<220>
<223> tumour antigen for melanoma

<400> 10
Ser Tyr Leu Asp Ser Gly Ile His Phe
1               5

<210> 11
<211> 12
<212> PRT
<213> Artificial Sequence

<220>
<223> tumour antigen for melanoma

<400> 11
Phe Ser Trp Ala Met Asp Leu Asp Pro Lys Gly Ala
1               5                   10

<210> 12
<211> 10
<212> PRT
<213> Artificial Sequence

<220>
<223> tumour antigen for melanoma

<400> 12
Ala Cys Asp Pro His Ser Gly His Phe Val
1               5                   10

<210> 13
<211> 9
<212> PRT
<213> Artificial Sequence

<220>
<223> tumour antigen for melanoma

<400> 13
Ala Val Cys Pro Trp Thr Trp Leu Arg
1               5

<210> 14
<211> 14
<212> PRT
<213> Artificial Sequence

<220>
<223> tumour antigen for colorectal carcinoma

<400> 14
Thr Leu Tyr Gln Asp Asp Thr Leu Thr Leu Gln Ala Ala Gly
1               5                   10

<210> 15
<211> 14
<212> PRT
<213> Artificial Sequence

<220>
<223> tumour antigen for colorectal carcinoma

<400> 15
Thr Leu Tyr Gln Asp Asp Thr Leu Thr Leu Gln Ala Ala Gly
1               5                   10

<210> 16
<211> 16
<212> PRT
<213> Artificial Sequence

<220>
<223> tumour antigen for myeloid leukemia

<400> 16
Thr Met Lys Gln Ile Cys Lys Lys Glu Ile Arg Arg Leu His Gln Tyr
1               5                   10                  15

<210> 17
<211> 10
<212> PRT
<213> Artificial Sequence

<220>
<223> tumour antigen for melanoma

<400> 17
Lys Ile Leu Asp Ala Val Val Ala Gln Lys
1               5                   10

<210> 18
<211> 9
<212> PRT
<213> Artificial Sequence

<220>
<223> tumour antigen for lung squamous CC

<400> 18
Glu Thr Val Ser Glu Gln Ser Asn Val
1               5

<210> 19
<211> 9
<212> PRT
<213> Artificial Sequence

<220>
<223> tumour antigen for acute lymphoblastic leukemia

<400> 19
Arg Ile Ala Glu Cys Ile Leu Gly Met
1               5

<210> 20

<211> 15
<212> PRT
<213> Artificial Sequence

<220>
<223> tumour antigen for acute lymphoblastic leukemia

<400> 20
Ile Gly Arg Ile Ala Glu Cys Ile Leu Gly Met Asn Pro Ser Arg
1               5                   10                  15

<210> 21
<211> 15
<212> PRT
<213> Artificial Sequence

<220>
<223> tumour antigen for acute lymphoblastic leukemia

<400> 21
Ile Gly Arg Ile Ala Glu Cys Ile Leu Gly Met Asn Pro Ser Arg
1               5                   10                  15

<210> 22
<211> 10
<212> PRT
<213> Artificial Sequence

<220>
<223> tumour antigen for acute myelogenous leukemia

<400> 22
Tyr Val Asp Phe Arg Glu Tyr Glu Tyr Tyr
1               5                   10

<210> 23
<211> 14
<212> PRT
<213> Artificial Sequence

<220>
<223> tumour antigen for melanoma

<400> 23
Met Ile Phe Glu Lys His Gly Phe Arg Arg Thr Thr Pro Pro
1               5                   10

<210> 24
<211> 10
<212> PRT
<213> Artificial Sequence

<220>
<223> tumour antigen for melanoma

<400> 24
Thr Leu Asp Trp Leu Leu Gln Thr Pro Lys
1               5                   10

<210> 25
<211> 9
<212> PRT
<213> Artificial Sequence

<220>
<223> tumour antigen for melanoma

<400> 25
Trp Arg Arg Ala Pro Ala Pro Gly Ala
1               5

<210> 26
<211> 9
<212> PRT
<213> Artificial Sequence

<220>
<223> tumour antigen for melanoma

<400> 26
Pro Val Thr Trp Arg Arg Ala Pro Ala
1               5

<210> 27
<211> 10
<212> PRT
<213> Artificial Sequence

<220>
<223> tumour antigen for renal cell carcinoma, for melanoma andr enal
      cell carcinoma

<400> 27
Ser Leu Phe Glu Gly Ile Asp Ile Tyr Thr
1               5                   10

<210> 28
<211> 9
<212> PRT
<213> Artificial Sequence

<220>
<223> tumour antigen for bladder tumour

<400> 28
Ala Glu Pro Ile Asn Ile Gln Thr Trp
1               5

<210> 29
<211> 11
<212> PRT
<213> Artificial Sequence

<220>
<223> tumour antigen for melanoma

<400> 29
Phe Leu Glu Gly Asn Glu Val Gly Lys Thr Tyr
1               5                   10

<210> 30
<211> 9
<212> PRT
<213> Artificial Sequence

<220>

<223> tumour antigen for non-small cell lung carcinoma

<400> 30
Phe Leu Asp Glu Phe Met Glu Gly Val
1               5

<210> 31
<211> 9
<212> PRT
<213> Artificial Sequence

<220>
<223> tumour antigen for melanoma

<400> 31
Glu Glu Lys Leu Ile Val Val Leu Phe
1               5

<210> 32
<211> 11
<212> PRT
<213> Artificial Sequence

<220>
<223> tumour antigen for melanoma

<400> 32
Ser Glu Leu Phe Arg Ser Gly Leu Asp Ser Tyr
1               5                   10

<210> 33
<211> 9
<212> PRT
<213> Artificial Sequence

<220>
<223> tumour antigen for melanoma

<400> 33
Phe Arg Ser Gly Leu Asp Ser Tyr Val
1               5

<210> 34
<211> 9
<212> PRT
<213> Artificial Sequence

<220>
<223> tumour antigen for melanoma

<400> 34
Glu Ala Phe Ile Gln Pro Ile Thr Arg
1               5

<210> 35
<211> 11
<212> PRT
<213> Artificial Sequence

<220>
<223> tumour antigen for melanoma

<400> 35

Arg Val Ile Lys Asn Ser Ile Arg Leu Thr Leu
1               5                   10

<210> 36
<211> 9
<212> PRT
<213> Artificial Sequence

<220>
<223> tumour antigen for melanoma

<400> 36
Lys Ile Asn Lys Asn Pro Lys Tyr Lys
1               5

<210> 37
<211> 8
<212> PRT
<213> Artificial Sequence

<220>
<223> tumour antigen for lung squamous cell carcinoma

<400> 37
Gln Gln Ile Thr Lys Thr Glu Val
1               5

<210> 38
<211> 10
<212> PRT
<213> Artificial Sequence

<220>
<223> tumour antigen for colorectal carcinoma

<400> 38
Ser Leu Tyr Lys Phe Ser Pro Phe Pro Leu
1               5                   10

<210> 39
<211> 9
<212> PRT
<213> Artificial Sequence

<220>
<223> tumour antigen for melanoma

<400> 39
Lys Glu Leu Glu Gly Ile Leu Leu Leu
1               5

<210> 40
<211> 9
<212> PRT
<213> Artificial Sequence

<220>
<223> tumour antigen for head and neck squamous cell carcinoma

<400> 40
Val Val Pro Cys Glu Pro Pro Glu Val
1               5

<210> 41
<211> 25
<212> PRT
<213> Artificial Sequence

<220>
<223> tumour antigen for promyelocytic leukemia

<400> 41
Asn Ser Asn His Val Ala Ser Gly Ala Gly Glu Ala Ala Ile Glu Thr
1               5                   10                  15
Gln Ser Ser Ser Ser Glu Glu Ile Val
            20                  25

<210> 42
<211> 10
<212> PRT
<213> Artificial Sequence

<220>
<223> tumour antigen for melanoma

<400> 42
Leu Leu Leu Asp Asp Leu Leu Val Ser Ile
1               5                   10

<210> 43
<211> 16
<212> PRT
<213> Artificial Sequence

<220>
<223> tumour antigen for melanoma

<400> 43
Pro Tyr Tyr Phe Ala Ala Glu Leu Pro Pro Arg Asn Leu Pro Glu Pro
1               5                   10                  15

<210> 44
<211> 9
<212> PRT
<213> Artificial Sequence

<220>
<223> tumour antigen for pancreatic adenocarcinoma

<400> 44
Val Val Val Gly Ala Val Gly Val Gly
1               5

<210> 45
<211> 10
<212> PRT
<213> Artificial Sequence

<220>
<223> tumour antigen for melanoma

<400> 45
Ile Leu Asp Thr Ala Gly Arg Glu Glu Tyr
1               5                   10

<210> 46
<211> 9
<212> PRT
<213> Artificial Sequence

<220>
<223> tumour antigen for melanoma

<400> 46
Arg Pro His Val Pro Glu Ser Ala Phe
1               5

<210> 47
<211> 9
<212> PRT
<213> Artificial Sequence

<220>
<223> tumour antigen for melanoma

<400> 47
Lys Ile Phe Ser Glu Val Thr Leu Lys
1               5

<210> 48
<211> 9
<212> PRT
<213> Artificial Sequence

<220>
<223> tumour antigen for melanoma

<400> 48
Ser His Glu Thr Val Ile Ile Glu Leu
1               5

<210> 49
<211> 10
<212> PRT
<213> Artificial Sequence

<220>
<223> tumour antigen for sarcoma

<400> 49
Gln Arg Pro Tyr Gly Tyr Asp Gln Ile Met
1               5                   10

<210> 50
<211> 9
<212> PRT
<213> Artificial Sequence

<220>
<223> tumour antigen for colorectal carcinoma

<400> 50
Arg Leu Ser Ser Cys Val Pro Val Ala
1               5

<210> 51
<211> 15

<212> PRT
<213> Artificial Sequence

<220>
<223> tumour antigen for melanoma

<400> 51
Gly Glu Leu Ile Gly Ile Leu Asn Ala Ala Lys Val Pro Ala Asp
1               5                   10                  15

<210> 52
<211> 9
<212> PRT
<213> Artificial Sequence

<220>
<223> shared tumour antigen

<400> 52
Ala Ala Arg Ala Val Phe Leu Ala Leu
1               5

<210> 53
<211> 8
<212> PRT
<213> Artificial Sequence

<220>
<223> shared tumour antigen

<400> 53
Tyr Arg Pro Arg Pro Arg Arg Tyr
1               5

<210> 54
<211> 9
<212> PRT
<213> Artificial Sequence

<220>
<223> shared tumour antigen

<400> 54
Tyr Tyr Trp Pro Arg Pro Arg Arg Tyr
1               5

<210> 55
<211> 10
<212> PRT
<213> Artificial Sequence

<220>
<223> shared tumour antigen

<400> 55
Val Leu Pro Asp Val Phe Ile Arg Cys Val
1               5                   10

<210> 56
<211> 9
<212> PRT
<213> Artificial Sequence

<220>
<223> shared tumour antigen

<400> 56
Met Leu Ala Val Ile Ser Cys Ala Val
1               5

<210> 57
<211> 9
<212> PRT
<213> Artificial Sequence

<220>
<223> shared tumour antigen

<400> 57
Arg Gln Lys Arg Ile Leu Val Asn Leu
1               5

<210> 58
<211> 9
<212> PRT
<213> Artificial Sequence

<220>
<223> shared tumour antigen

<400> 58
Asn Tyr Asn Asn Phe Tyr Arg Phe Leu
1               5

<210> 59
<211> 10
<212> PRT
<213> Artificial Sequence

<220>
<223> shared tumour antigen

<400> 59
Glu Tyr Ser Lys Glu Cys Leu Lys Glu Phe
1               5                   10

<210> 60
<211> 9
<212> PRT
<213> Artificial Sequence

<220>
<223> shared tumour antigen

<400> 60
Glu Tyr Leu Ser Leu Ser Asp Lys Ile
1               5

<210> 61
<211> 11
<212> PRT
<213> Artificial Sequence

<220>
<223> shared tumour antigen

<400> 61
Met Leu Met Ala Gln Glu Ala Leu Ala Phe Leu
1               5                   10

<210> 62
<211> 9
<212> PRT
<213> Artificial Sequence

<220>
<223> shared tumour antigen

<400> 62
Ser Leu Leu Met Trp Ile Thr Gln Cys
1               5

<210> 63
<211> 10
<212> PRT
<213> Artificial Sequence

<220>
<223> shared tumour antigen

<400> 63
Leu Ala Ala Gln Glu Arg Arg Val Pro Arg
1               5                   10

<210> 64
<211> 9
<212> PRT
<213> Artificial Sequence

<220>
<223> shared tumour antigen

<400> 64
Glu Leu Val Arg Arg Ile Leu Ser Arg
1               5

<210> 65
<211> 9
<212> PRT
<213> Artificial Sequence

<220>
<223> shared tumour antigen

<400> 65
Ala Pro Arg Gly Val Arg Met Ala Val
1               5

<210> 66
<211> 14
<212> PRT
<213> Artificial Sequence

<220>
<223> shared tumour antigen

<400> 66
Ser Leu Leu Met Trp Ile Thr Gln Cys Phe Leu Pro Val Phe
1               5                   10

<210> 67
<211> 20
<212> PRT
<213> Artificial Sequence

<220>
<223> shared tumour antigen

<400> 67
Gln Gly Ala Met Leu Ala Ala Gln Glu Arg Arg Val Pro Arg Ala Ala
1               5                   10                  15
Glu Val Pro Arg
            20

<210> 68
<211> 18
<212> PRT
<213> Artificial Sequence

<220>
<223> shared tumour antigen

<400> 68
Ala Ala Asp His Arg Gln Leu Gln Leu Ser Ile Ser Ser Cys Leu Gln
1               5                   10                  15
Gln Leu


<210> 69
<211> 22
<212> PRT
<213> Artificial Sequence

<220>
<223> shared tumour antigen

<400> 69
Cys Leu Ser Arg Arg Pro Trp Lys Arg Ser Trp Ser Ala Gly Ser Cys
1               5                   10                  15
Pro Gly Met Pro His Leu
            20

<210> 70
<211> 22
<212> PRT
<213> Artificial Sequence

<220>
<223> shared tumour antigen

<400> 70
Cys Leu Ser Arg Arg Pro Trp Lys Arg Ser Trp Ser Ala Gly Ser Cys
1               5                   10                  15
Pro Gly Met Pro His Leu
            20

<210> 71
<211> 13
<212> PRT
<213> Artificial Sequence

<220>

<223> shared tumour antigen

<400> 71
Ile Leu Ser Arg Asp Ala Ala Pro Leu Pro Arg Pro Gly
1               5                   10

<210> 72
<211> 14
<212> PRT
<213> Artificial Sequence

<220>
<223> shared tumour antigen

<400> 72
Ala Gly Ala Thr Gly Gly Arg Gly Pro Arg Gly Ala Gly Ala
1               5                   10

<210> 73
<211> 9
<212> PRT
<213> Artificial Sequence

<220>
<223> shared tumour antigen

<400> 73
Glu Ala Asp Pro Thr Gly His Ser Tyr
1               5

<210> 74
<211> 9
<212> PRT
<213> Artificial Sequence

<220>
<223> shared tumour antigen

<400> 74
Lys Val Leu Glu Tyr Val Ile Lys Val
1               5

<210> 75
<211> 9
<212> PRT
<213> Artificial Sequence

<220>
<223> shared tumour antigen

<400> 75
Ser Leu Phe Arg Ala Val Ile Thr Lys
1               5

<210> 76
<211> 10
<212> PRT
<213> Artificial Sequence

<220>
<223> shared tumour antigen

<400> 76

Glu Val Tyr Asp Gly Arg Glu His Ser Ala
1               5                   10

<210> 77
<211> 9
<212> PRT
<213> Artificial Sequence

<220>
<223> shared tumour antigen

<400> 77
Arg Val Arg Phe Phe Phe Pro Ser Leu
1               5

<210> 78
<211> 9
<212> PRT
<213> Artificial Sequence

<220>
<223> shared tumour antigen

<400> 78
Glu Ala Asp Pro Thr Gly His Ser Tyr
1               5

<210> 79
<211> 10
<212> PRT
<213> Artificial Sequence

<220>
<223> shared tumour antigen

<400> 79
Arg Glu Pro Val Thr Lys Ala Glu Met Leu
1               5                   10

<210> 80
<211> 9
<212> PRT
<213> Artificial Sequence

<220>
<223> shared tumour antigen

<400> 80
Asp Pro Ala Arg Tyr Glu Phe Leu Trp
1               5

<210> 81
<211> 11
<212> PRT
<213> Artificial Sequence

<220>
<223> shared tumour antigen

<400> 81
Ile Thr Lys Lys Val Ala Asp Leu Val Gly Phe
1               5                   10

<210> 82
<211> 9
<212> PRT
<213> Artificial Sequence

<220>
<223> shared tumour antigen

<400> 82
Ser Ala Phe Pro Thr Thr Ile Asn Phe
1               5

<210> 83
<211> 9
<212> PRT
<213> Artificial Sequence

<220>
<223> shared tumour antigen

<400> 83
Ser Ala Tyr Gly Glu Pro Arg Lys Leu
1               5

<210> 84
<211> 9
<212> PRT
<213> Artificial Sequence

<220>
<223> shared tumour antigen

<400> 84
Ser Ala Tyr Gly Glu Pro Arg Lys Leu
1               5

<210> 85
<211> 15
<212> PRT
<213> Artificial Sequence

<220>
<223> shared tumour antigen

<400> 85
Thr Ser Cys Ile Leu Glu Ser Leu Phe Arg Ala Val Ile Thr Lys
1               5                   10                  15

<210> 86
<211> 15
<212> PRT
<213> Artificial Sequence

<220>
<223> shared tumour antigen

<400> 86
Pro Arg Ala Leu Ala Glu Thr Ser Tyr Val Lys Val Leu Glu Tyr
1               5                   10                  15

<210> 87
<211> 16
<212> PRT

<213> Artificial Sequence

<220>
<223> shared tumour antigen

<400> 87
Phe Leu Leu Leu Lys Tyr Arg Ala Arg Glu Pro Val Thr Lys Ala Glu
1               5                   10                  15

<210> 88
<211> 12
<212> PRT
<213> Artificial Sequence

<220>
<223> shared tumour antigen

<400> 88
Glu Tyr Val Ile Lys Val Ser Ala Arg Val Arg Phe
1               5                   10

<210> 89
<211> 10
<212> PRT
<213> Artificial Sequence

<220>
<223> shared tumour antigen

<400> 89
Tyr Leu Gln Leu Val Phe Gly Ile Glu Val
1               5                   10

<210> 90
<211> 9
<212> PRT
<213> Artificial Sequence

<220>
<223> shared tumour antigen

<400> 90
Glu Tyr Leu Gln Leu Val Phe Gly Ile
1               5

<210> 91
<211> 10
<212> PRT
<213> Artificial Sequence

<220>
<223> shared tumour antigen

<400> 91
Arg Glu Pro Val Thr Lys Ala Glu Met Leu
1               5                   10

<210> 92
<211> 9
<212> PRT
<213> Artificial Sequence

<220>
<223> shared tumour antigen

<400> 92
Glu Gly Asp Cys Ala Pro Glu Glu Lys
1               5

<210> 93
<211> 14
<212> PRT
<213> Artificial Sequence

<220>
<223> shared tumour antigen

<400> 93
Leu Leu Lys Tyr Arg Ala Arg Glu Pro Val Thr Lys Ala Glu
1               5                   10

<210> 94
<211> 9
<212> PRT
<213> Artificial Sequence

<220>
<223> shared tumour antigen

<400> 94
Glu Val Asp Pro Ile Gly His Leu Tyr
1               5

<210> 95
<211> 9
<212> PRT
<213> Artificial Sequence

<220>
<223> shared tumour antigen

<400> 95
Phe Leu Trp Gly Pro Arg Ala Leu Val
1               5

<210> 96
<211> 9
<212> PRT
<213> Artificial Sequence

<220>
<223> shared tumour antigen

<400> 96
Lys Val Ala Glu Leu Val His Phe Leu
1               5

<210> 97
<211> 9
<212> PRT
<213> Artificial Sequence

<220>
<223> shared tumour antigen

<400> 97
Thr Phe Pro Asp Leu Glu Ser Glu Phe
1               5

<210> 98
<211> 9
<212> PRT
<213> Artificial Sequence

<220>
<223> shared tumour antigen

<400> 98
Val Ala Glu Leu Val His Phe Leu Leu
1               5

<210> 99
<211> 10
<212> PRT
<213> Artificial Sequence

<220>
<223> shared tumour antigen

<400> 99
Met Glu Val Asp Pro Ile Gly His Leu Tyr
1               5                   10

<210> 100
<211> 9
<212> PRT
<213> Artificial Sequence

<220>
<223> shared tumour antigen

<400> 100
Glu Val Asp Pro Ile Gly His Leu Tyr
1               5

<210> 101
<211> 10
<212> PRT
<213> Artificial Sequence

<220>
<223> shared tumour antigen

<400> 101
Arg Glu Pro Val Thr Lys Ala Glu Met Leu
1               5                   10

<210> 102
<211> 9
<212> PRT
<213> Artificial Sequence

<220>
<223> shared tumour antigen

<400> 102
Ala Glu Leu Val His Phe Leu Leu Leu

1               5

<210> 103
<211> 10
<212> PRT
<213> Artificial Sequence

<220>
<223> shared tumour antigen

<400> 103
Met Glu Val Asp Pro Ile Gly His Leu Tyr
1               5                   10

<210> 104
<211> 9
<212> PRT
<213> Artificial Sequence

<220>
<223> shared tumour antigen

<400> 104
Trp Gln Tyr Phe Phe Pro Val Ile Phe
1               5

<210> 105
<211> 9
<212> PRT
<213> Artificial Sequence

<220>
<223> shared tumour antigen

<400> 105
Glu Gly Asp Cys Ala Pro Glu Glu Lys
1               5

<210> 106
<211> 16
<212> PRT
<213> Artificial Sequence

<220>
<223> shared tumour antigen

<400> 106
Lys Lys Leu Leu Thr Gln His Phe Val Gln Glu Asn Tyr Leu Glu Tyr
1               5                   10                  15

<210> 107
<211> 16
<212> PRT
<213> Artificial Sequence

<220>
<223> shared tumour antigen

<400> 107
Lys Lys Leu Leu Thr Gln His Phe Val Gln Glu Asn Tyr Leu Glu Tyr
1               5                   10                  15

<210> 108
<211> 16
<212> PRT
<213> Artificial Sequence

<220>
<223> shared tumour antigen

<400> 108
Ala Cys Tyr Glu Phe Leu Trp Gly Pro Arg Ala Leu Val Glu Thr Ser
1               5                   10                  15

<210> 109
<211> 12
<212> PRT
<213> Artificial Sequence

<220>
<223> shared tumour antigen

<400> 109
Val Ile Phe Ser Lys Ala Ser Ser Ser Leu Gln Leu
1               5                   10

<210> 110
<211> 12
<212> PRT
<213> Artificial Sequence

<220>
<223> shared tumour antigen

<400> 110
Val Ile Phe Ser Lys Ala Ser Ser Ser Leu Gln Leu
1               5                   10

<210> 111
<211> 15
<212> PRT
<213> Artificial Sequence

<220>
<223> shared tumour antigen

<400> 111
Gly Asp Asn Gln Ile Met Pro Lys Ala Gly Leu Leu Ile Ile Val
1               5                   10                  15

<210> 112
<211> 15
<212> PRT
<213> Artificial Sequence

<220>
<223> shared tumour antigen

<400> 112
Thr Ser Tyr Val Lys Val Leu His His Met Val Lys Ile Ser Gly

1               5                   10                  15

<210> 113
<211> 16
<212> PRT
<213> Artificial Sequence

<220>
<223> shared tumour antigen

<400> 113
Arg Lys Val Ala Glu Leu Val His Phe Leu Leu Leu Lys Tyr Arg Ala
1               5                   10                  15

<210> 114
<211> 16
<212> PRT
<213> Artificial Sequence

<220>
<223> shared tumour antigen

<400> 114
Phe Leu Leu Leu Lys Tyr Arg Ala Arg Glu Pro Val Thr Lys Ala Glu
1               5                   10                  15

<210> 115
<211> 9
<212> PRT
<213> Artificial Sequence

<220>
<223> shared tumour antigen

<400> 115
Glu Val Asp Pro Ala Ser Asn Thr Tyr
1               5

<210> 116
<211> 10
<212> PRT
<213> Artificial Sequence

<220>
<223> shared tumour antigen

<400> 116
Gly Val Tyr Asp Gly Arg Glu His Thr Val
1               5                   10

<210> 117
<211> 9
<212> PRT
<213> Artificial Sequence

<220>
<223> shared tumour antigen

<400> 117

Asn Tyr Lys Arg Cys Phe Pro Val Ile
1               5

<210> 118
<211> 8
<212> PRT
<213> Artificial Sequence

<220>
<223> shared tumour antigen

<400> 118
Ser Glu Ser Leu Lys Met Ile Phe
1               5

<210> 119
<211> 9
<212> PRT
<213> Artificial Sequence

<220>
<223> shared tumour antigen

<400> 119
Met Val Lys Ile Ser Gly Gly Pro Arg
1               5

<210> 120
<211> 9
<212> PRT
<213> Artificial Sequence

<220>
<223> shared tumour antigen

<400> 120
Glu Val Asp Pro Ile Gly His Val Tyr
1               5

<210> 121
<211> 10
<212> PRT
<213> Artificial Sequence

<220>
<223> shared tumour antigen

<400> 121
Arg Glu Pro Val Thr Lys Ala Glu Met Leu
1               5                   10

<210> 122
<211> 9
<212> PRT
<213> Artificial Sequence

<220>
<223> shared tumour antigen

<400> 122
Glu Gly Asp Cys Ala Pro Glu Glu Lys
1               5

<210> 123
<211> 9
<212> PRT
<213> Artificial Sequence

<220>
<223> shared tumour antigen

<400> 123
Ile Ser Gly Gly Pro Arg Ile Ser Tyr
1               5

<210> 124
<211> 14
<212> PRT
<213> Artificial Sequence

<220>
<223> shared tumour antigen

<400> 124
Leu Leu Lys Tyr Arg Ala Arg Glu Pro Val Thr Lys Ala Glu
1               5                   10

<210> 125
<211> 9
<212> PRT
<213> Artificial Sequence

<220>
<223> shared tumour antigen

<400> 125
Ala Leu Ser Val Met Gly Val Tyr Val
1               5

<210> 126
<211> 9
<212> PRT
<213> Artificial Sequence

<220>
<223> shared tumour antigen

<400> 126
Gly Leu Tyr Asp Gly Met Glu His Leu
1               5

<210> 127
<211> 9
<212> PRT
<213> Artificial Sequence

<220>
<223> shared tumour antigen

<400> 127
Asp Pro Ala Arg Tyr Glu Phe Leu Trp
1               5

<210> 128
<211> 9
<212> PRT

<213> Artificial Sequence

<220>
<223> shared tumour antigen

<400> 128
Phe Leu Trp Gly Pro Arg Ala Leu Val
1               5

<210> 129
<211> 9
<212> PRT
<213> Artificial Sequence

<220>
<223> shared tumour antigen

<400> 129
Val Arg Ile Gly His Leu Tyr Ile Leu
1               5

<210> 130
<211> 9
<212> PRT
<213> Artificial Sequence

<220>
<223> shared tumour antigen

<400> 130
Glu Gly Asp Cys Ala Pro Glu Glu Lys
1               5

<210> 131
<211> 15
<212> PRT
<213> Artificial Sequence

<220>
<223> shared tumour antigen

<400> 131
Arg Glu Pro Phe Thr Lys Ala Glu Met Leu Gly Ser Val Ile Arg
1               5                   10                  15

<210> 132
<211> 14
<212> PRT
<213> Artificial Sequence

<220>
<223> shared tumour antigen

<400> 132
Ala Glu Leu Val His Phe Leu Leu Leu Lys Tyr Arg Ala Arg
1               5                   10

<210> 133
<211> 10
<212> PRT
<213> Artificial Sequence

<220>

<223> shared tumour antigen

<400> 133
Leu Leu Phe Gly Leu Ala Leu Ile Glu Val
1               5                   10

<210> 134
<211> 9
<212> PRT
<213> Artificial Sequence

<220>
<223> shared tumour antigen

<400> 134
Ala Leu Lys Asp Val Glu Glu Arg Val
1               5

<210> 135
<211> 9
<212> PRT
<213> Artificial Sequence

<220>
<223> shared tumour antigen

<400> 135
Ser Glu Ser Ile Lys Lys Lys Val Leu
1               5

<210> 136
<211> 20
<212> PRT
<213> Artificial Sequence

<220>
<223> shared tumour antigen

<400> 136
Pro Asp Thr Arg Pro Ala Pro Gly Ser Thr Ala Pro Pro Ala His Gly
1               5                   10                  15
Val Thr Ser Ala
            20

<210> 137
<211> 9
<212> PRT
<213> Artificial Sequence

<220>
<223> shared tumour antigen

<400> 137
Gln Gly Gln His Phe Leu Gln Lys Val
1               5

<210> 138
<211> 9
<212> PRT
<213> Artificial Sequence

<220>
<223> shared tumour antigen

<400> 138
Ser Leu Leu Met Trp Ile Thr Gln Cys
1               5

<210> 139
<211> 11
<212> PRT
<213> Artificial Sequence

<220>
<223> shared tumour antigen

<400> 139
Met Leu Met Ala Gln Glu Ala Leu Ala Phe Leu
1               5                   10

<210> 140
<211> 10
<212> PRT
<213> Artificial Sequence

<220>
<223> shared tumour antigen

<400> 140
Ala Ser Gly Pro Gly Gly Gly Ala Pro Arg
1               5                   10

<210> 141
<211> 10
<212> PRT
<213> Artificial Sequence

<220>
<223> shared tumour antigen

<400> 141
Leu Ala Ala Gln Glu Arg Arg Val Pro Arg
1               5                   10

<210> 142
<211> 10
<212> PRT
<213> Artificial Sequence

<220>
<223> shared tumour antigen

<400> 142
Thr Val Ser Gly Asn Ile Leu Thr Ile Arg
1               5                   10

<210> 143
<211> 13
<212> PRT
<213> Artificial Sequence

<220>
<223> shared tumour antigen

<400> 143
Ala Pro Arg Gly Pro His Gly Gly Ala Ala Ser Gly Leu

1                 5                   10

<210> 144
<211> 9
<212> PRT
<213> Artificial Sequence

<220>
<223> shared tumour antigen

<400> 144
Met Pro Phe Ala Thr Pro Met Glu Ala
1                 5

<210> 145
<211> 12
<212> PRT
<213> Artificial Sequence

<220>
<223> shared tumour antigen

<400> 145
Lys Glu Phe Thr Val Ser Gly Asn Ile Leu Thr Ile
1                 5                   10

<210> 146
<211> 9
<212> PRT
<213> Artificial Sequence

<220>
<223> shared tumour antigen

<400> 146
Met Pro Phe Ala Thr Pro Met Glu Ala
1                 5

<210> 147
<211> 9
<212> PRT
<213> Artificial Sequence

<220>
<223> shared tumour antigen

<400> 147
Leu Ala Met Pro Phe Ala Thr Pro Met
1                 5

<210> 148
<211> 9
<212> PRT
<213> Artificial Sequence

<220>
<223> shared tumour antigen

<400> 148
Ala Arg Gly Pro Glu Ser Arg Leu Leu
1                 5

<210> 149

<211> 14
<212> PRT
<213> Artificial Sequence

<220>
<223> shared tumour antigen

<400> 149
Ser Leu Leu Met Trp Ile Thr Gln Cys Phe Leu Pro Val Phe
1               5                   10

<210> 150
<211> 25
<212> PRT
<213> Artificial Sequence

<220>
<223> shared tumour antigen

<400> 150
Leu Leu Glu Phe Tyr Leu Ala Met Pro Phe Ala Thr Pro Met Glu Ala
1               5                   10                  15
Glu Leu Ala Arg Arg Ser Leu Ala Gln
            20                  25

<210> 151
<211> 25
<212> PRT
<213> Artificial Sequence

<220>
<223> shared tumour antigen

<400> 151
Leu Leu Glu Phe Tyr Leu Ala Met Pro Phe Ala Thr Pro Met Glu Ala
1               5                   10                  15
Glu Leu Ala Arg Arg Ser Leu Ala Gln
            20                  25

<210> 152
<211> 12
<212> PRT
<213> Artificial Sequence

<220>
<223> shared tumour antigen

<400> 152
Glu Phe Tyr Leu Ala Met Pro Phe Ala Thr Pro Met
1               5                   10

<210> 153
<211> 12
<212> PRT
<213> Artificial Sequence

<220>
<223> shared tumour antigen

<400> 153
Arg Leu Leu Glu Phe Tyr Leu Ala Met Pro Phe Ala
1               5                   10

<210> 154
<211> 20
<212> PRT
<213> Artificial Sequence

<220>
<223> shared tumour antigen

<400> 154
Gln Gly Ala Met Leu Ala Ala Gln Glu Arg Arg Val Pro Arg Ala Ala
1               5                   10                  15
Glu Val Pro Arg
            20

<210> 155
<211> 20
<212> PRT
<213> Artificial Sequence

<220>
<223> shared tumour antigen

<400> 155
Pro Gly Val Leu Leu Lys Glu Phe Thr Val Ser Gly Asn Ile Leu Thr
1               5                   10                  15
Ile Arg Leu Thr
            20

<210> 156
<211> 10
<212> PRT
<213> Artificial Sequence

<220>
<223> shared tumour antigen

<400> 156
Val Leu Leu Lys Glu Phe Thr Val Ser Gly
1               5                   10

<210> 157
<211> 18
<212> PRT
<213> Artificial Sequence

<220>
<223> shared tumour antigen

<400> 157
Ala Ala Asp His Arg Gln Leu Gln Leu Ser Ile Ser Ser Cys Leu Gln
1               5                   10                  15
Gln Leu


<210> 158
<211> 25
<212> PRT
<213> Artificial Sequence

<220>
<223> shared tumour antigen

<400> 158

Leu Leu Glu Phe Tyr Leu Ala Met Pro Phe Ala Thr Pro Met Glu Ala
1               5                   10                  15
Glu Leu Ala Arg Arg Ser Leu Ala Gln
            20                  25

<210> 159
<211> 15
<212> PRT
<213> Artificial Sequence

<220>
<223> shared tumour antigen

<400> 159
Leu Lys Glu Phe Thr Val Ser Gly Asn Ile Leu Thr Ile Arg Leu
1               5                   10                  15

<210> 160
<211> 25
<212> PRT
<213> Artificial Sequence

<220>
<223> shared tumour antigen

<400> 160
Pro Gly Val Leu Leu Lys Glu Phe Thr Val Ser Gly Asn Ile Leu Thr
1               5                   10                  15
Ile Arg Leu Thr Ala Ala Asp His Arg
            20                  25

<210> 161
<211> 25
<212> PRT
<213> Artificial Sequence

<220>
<223> shared tumour antigen

<400> 161
Leu Leu Glu Phe Tyr Leu Ala Met Pro Phe Ala Thr Pro Met Glu Ala
1               5                   10                  15
Glu Leu Ala Arg Arg Ser Leu Ala Gln
            20                  25

<210> 162
<211> 14
<212> PRT
<213> Artificial Sequence

<220>
<223> shared tumour antigen

<400> 162
Ala Gly Ala Thr Gly Gly Arg Gly Pro Arg Gly Ala Gly Ala
1               5                   10

<210> 163
<211> 9
<212> PRT
<213> Artificial Sequence

<220>

<223> shared tumour antigen

<400> 163
Leu Tyr Ala Thr Val Ile His Asp Ile
1               5

<210> 164
<211> 9
<212> PRT
<213> Artificial Sequence

<220>
<223> shared tumour antigen

<400> 164
Ile Leu Asp Ser Ser Glu Glu Asp Lys
1               5

<210> 165
<211> 9
<212> PRT
<213> Artificial Sequence

<220>
<223> shared tumour antigen

<400> 165
Lys Ala Ser Glu Lys Ile Phe Tyr Val
1               5

<210> 166
<211> 16
<212> PRT
<213> Artificial Sequence

<220>
<223> shared tumour antigen

<400> 166
Glu Lys Ile Gln Lys Ala Phe Asp Asp Ile Ala Lys Tyr Phe Ser Lys
1               5                   10                  15

<210> 167
<211> 18
<212> PRT
<213> Artificial Sequence

<220>
<223> shared tumour antigen

<400> 167
Trp Glu Lys Met Lys Ala Ser Glu Lys Ile Phe Tyr Val Tyr Met Lys
1               5                   10                  15
Arg Lys

<210> 168
<211> 15
<212> PRT
<213> Artificial Sequence

<220>
<223> shared tumour antigen

<400> 168
Lys Ile Phe Tyr Val Tyr Met Lys Arg Lys Tyr Glu Ala Met Thr
1               5                   10                  15

<210> 169
<211> 14
<212> PRT
<213> Artificial Sequence

<220>
<223> shared tumour antigen

<400> 169
Lys Ile Phe Tyr Val Tyr Met Lys Arg Lys Tyr Glu Ala Met
1               5                   10

<210> 170
<211> 20
<212> PRT
<213> Artificial Sequence

<220>
<223> shared tumour antigen

<400> 170
Ile Asn Lys Thr Ser Gly Pro Lys Arg Gly Lys His Ala Trp Thr His
1               5                   10                  15
Arg Leu Arg Glu
            20

<210> 171
<211> 20
<212> PRT
<213> Artificial Sequence

<220>
<223> shared tumour antigen

<400> 171
Tyr Phe Ser Lys Lys Glu Trp Glu Lys Met Lys Ser Ser Glu Lys Ile
1               5                   10                  15
Val Tyr Val Tyr
            20

<210> 172
<211> 20
<212> PRT
<213> Artificial Sequence

<220>
<223> shared tumour antigen

<400> 172
Met Lys Leu Asn Tyr Glu Val Met Thr Lys Leu Gly Phe Lys Val Thr
1               5                   10                  15
Leu Pro Pro Phe
            20

<210> 173
<211> 20

<212> PRT
<213> Artificial Sequence

<220>
<223> shared tumour antigen

<400> 173
Lys His Ala Trp Thr His Arg Leu Arg Glu Arg Lys Gln Leu Val Val
1               5                   10                  15
Tyr Glu Glu Ile
            20

<210> 174
<211> 20
<212> PRT
<213> Artificial Sequence

<220>
<223> shared tumour antigen

<400> 174
Leu Gly Phe Lys Val Thr Leu Pro Pro Phe Met Arg Ser Lys Arg Ala
1               5                   10                  15
Ala Asp Phe His
            20

<210> 175
<211> 20
<212> PRT
<213> Artificial Sequence

<220>
<223> shared tumour antigen

<400> 175
Lys Ser Ser Glu Lys Ile Val Tyr Val Tyr Met Lys Leu Asn Tyr Glu
1               5                   10                  15
Val Met Thr Lys
            20

<210> 176
<211> 20
<212> PRT
<213> Artificial Sequence

<220>
<223> shared tumour antigen

<400> 176
Lys His Ala Trp Thr His Arg Leu Arg Glu Arg Lys Gln Leu Val Val
1               5                   10                  15
Tyr Glu Glu Ile
            20

<210> 177
<211> 9
<212> PRT
<213> Artificial Sequence

<220>
<223> shared tumour antigen

<400> 177

Ser Leu Gly Trp Leu Phe Leu Leu Leu
1               5

<210> 178
<211> 9
<212> PRT
<213> Artificial Sequence

<220>
<223> shared tumour antigen

<400> 178
Leu Ser Arg Leu Ser Asn Arg Leu Leu
1               5

<210> 179
<211> 9
<212> PRT
<213> Artificial Sequence

<220>
<223> shared tumour antigen

<400> 179
Leu Ser Arg Leu Ser Asn Arg Leu Leu
1               5

<210> 180
<211> 15
<212> PRT
<213> Artificial Sequence

<220>
<223> shared tumour antigen

<400> 180
Cys Glu Phe His Ala Cys Trp Pro Ala Phe Thr Val Leu Gly Glu
1               5                   10                  15

<210> 181
<211> 15
<212> PRT
<213> Artificial Sequence

<220>
<223> shared tumour antigen

<400> 181
Cys Glu Phe His Ala Cys Trp Pro Ala Phe Thr Val Leu Gly Glu
1               5                   10                  15

<210> 182
<211> 15
<212> PRT
<213> Artificial Sequence

<220>
<223> shared tumour antigen

<400> 182
Cys Glu Phe His Ala Cys Trp Pro Ala Phe Thr Val Leu Gly Glu
1               5                   10                  15

<210> 183
<211> 10
<212> PRT
<213> Artificial Sequence

<220>
<223> shared tumour antigen

<400> 183
Glu Val Ile Ser Cys Lys Leu Ile Lys Arg
1               5                   10

<210> 184
<211> 17
<212> PRT
<213> Artificial Sequence

<220>
<223> shared tumour antigen

<400> 184
Cys Ala Thr Trp Lys Val Ile Cys Lys Ser Cys Ile Ser Gln Thr Pro
1               5                   10                  15
Gly


<210> 185
<211> 9
<212> PRT
<213> Artificial Sequence

<220>
<223> tumour differentiation antigen

<400> 185
Tyr Leu Ser Gly Ala Asn Leu Asn Leu
1               5

<210> 186
<211> 9
<212> PRT
<213> Artificial Sequence

<220>
<223> tumour differentiation antigen

<400> 186
Ile Met Ile Gly Val Leu Val Gly Val
1               5

<210> 187
<211> 9
<212> PRT
<213> Artificial Sequence

<220>
<223> tumour differentiation antigen

<400> 187
Gly Val Leu Val Gly Val Ala Leu Ile
1               5

<210> 188

<211> 9
<212> PRT
<213> Artificial Sequence

<220>
<223> tumour differentiation antigen

<400> 188
His Leu Phe Gly Tyr Ser Trp Tyr Lys
1               5

<210> 189
<211> 10
<212> PRT
<213> Artificial Sequence

<220>
<223> tumour differentiation antigen

<400> 189
Gln Tyr Ser Trp Phe Val Asn Gly Thr Phe
1               5                   10

<210> 190
<211> 9
<212> PRT
<213> Artificial Sequence

<220>
<223> tumour differentiation antigen

<400> 190
Thr Tyr Ala Cys Phe Val Ser Asn Leu
1               5

<210> 191
<211> 15
<212> PRT
<213> Artificial Sequence

<220>
<223> tumour differentiation antigen

<400> 191
Ala Tyr Val Cys Gly Ile Gln Asn Ser Val Ser Ala Asn Arg Ser
1               5                   10                  15

<210> 192
<211> 25
<212> PRT
<213> Artificial Sequence

<220>
<223> tumour differentiation antigen

<400> 192
Asp Thr Gly Phe Tyr Thr Leu His Val Ile Lys Ser Asp Leu Val Asn
1               5                   10                  15
Glu Glu Ala Thr Gly Gln Phe Arg Val
            20                  25

<210> 193
<211> 15

<212> PRT
<213> Artificial Sequence

<220>
<223> tumour differentiation antigen

<400> 193
Tyr Ser Trp Arg Ile Asn Gly Ile Pro Gln Gln His Thr Gln Val
1               5                   10                  15

<210> 194
<211> 13
<212> PRT
<213> Artificial Sequence

<220>
<223> tumour differentiation antigen

<400> 194
Thr Tyr Tyr Arg Pro Gly Val Asn Leu Ser Leu Ser Cys
1               5                   10

<210> 195
<211> 13
<212> PRT
<213> Artificial Sequence

<220>
<223> tumour differentiation antigen

<400> 195
Glu Ile Ile Tyr Pro Asn Ala Ser Leu Leu Ile Gln Asn
1               5                   10

<210> 196
<211> 15
<212> PRT
<213> Artificial Sequence

<220>
<223> tumour differentiation antigen

<400> 196
Tyr Ala Cys Phe Val Ser Asn Leu Ala Thr Gly Arg Asn Asn Ser
1               5                   10                  15

<210> 197
<211> 13
<212> PRT
<213> Artificial Sequence

<220>
<223> tumour differentiation antigen

<400> 197
Leu Trp Trp Val Asn Asn Gln Ser Leu Pro Val Ser Pro
1               5                   10

<210> 198
<211> 13
<212> PRT
<213> Artificial Sequence

<220>
<223> tumour differentiation antigen

<400> 198
Leu Trp Trp Val Asn Asn Gln Ser Leu Pro Val Ser Pro
1               5                   10

<210> 199
<211> 13
<212> PRT
<213> Artificial Sequence

<220>
<223> tumour differentiation antigen

<400> 199
Leu Trp Trp Val Asn Asn Gln Ser Leu Pro Val Ser Pro
1               5                   10

<210> 200
<211> 13
<212> PRT
<213> Artificial Sequence

<220>
<223> tumour differentiation antigen

<400> 200
Glu Ile Ile Tyr Pro Asn Ala Ser Leu Leu Ile Gln Asn
1               5                   10

<210> 201
<211> 13
<212> PRT
<213> Artificial Sequence

<220>
<223> tumour differentiation antigen

<400> 201
Asn Ser Ile Val Lys Ser Ile Thr Val Ser Ala Ser Gly
1               5                   10

<210> 202
<211> 9
<212> PRT
<213> Artificial Sequence

<220>
<223> tumour differentiation antigen

<400> 202
Lys Thr Trp Gly Gln Tyr Trp Gln Val
1               5

<210> 203
<211> 10
<212> PRT
<213> Artificial Sequence

<220>
<223> tumour differentiation antigen

<400> 203
Ala Met Leu Gly Thr His Thr Met Glu Val
1               5                   10

<210> 204
<211> 9
<212> PRT
<213> Artificial Sequence

<220>
<223> tumour differentiation antigen

<400> 204
Ile Thr Asp Gln Val Pro Phe Ser Val
1               5

<210> 205
<211> 9
<212> PRT
<213> Artificial Sequence

<220>
<223> tumour differentiation antigen

<400> 205
Tyr Leu Glu Pro Gly Pro Val Thr Ala
1               5

<210> 206
<211> 10
<212> PRT
<213> Artificial Sequence

<220>
<223> tumour differentiation antigen

<400> 206
Leu Leu Asp Gly Thr Ala Thr Leu Arg Leu
1               5                   10

<210> 207
<211> 10
<212> PRT
<213> Artificial Sequence

<220>
<223> tumour differentiation antigen

<400> 207
Val Leu Tyr Arg Tyr Gly Ser Phe Ser Val
1               5                   10

<210> 208
<211> 10
<212> PRT
<213> Artificial Sequence

<220>
<223> tumour differentiation antigen

<400> 208
Ser Leu Ala Asp Thr Asn Ser Leu Ala Val
1               5                   10

<210> 209
<211> 9
<212> PRT
<213> Artificial Sequence

<220>
<223> tumour differentiation antigen

<400> 209
Arg Leu Met Lys Gln Asp Phe Ser Val
1               5

<210> 210
<211> 9
<212> PRT
<213> Artificial Sequence

<220>
<223> tumour differentiation antigen

<400> 210
Arg Leu Pro Arg Ile Phe Cys Ser Cys
1               5

<210> 211
<211> 9
<212> PRT
<213> Artificial Sequence

<220>
<223> tumour differentiation antigen

<400> 211
Leu Ile Tyr Arg Arg Arg Leu Met Lys
1               5

<210> 212
<211> 9
<212> PRT
<213> Artificial Sequence

<220>
<223> tumour differentiation antigen

<400> 212
Ala Leu Leu Ala Val Gly Ala Thr Lys
1               5

<210> 213
<211> 10
<212> PRT
<213> Artificial Sequence

<220>
<223> tumour differentiation antigen

<400> 213
Ile Ala Leu Asn Phe Pro Gly Ser Gln Lys
1               5                   10

<210> 214
<211> 9

<212> PRT
<213> Artificial Sequence

<220>
<223> tumour differentiation antigen

<400> 214
Ala Leu Asn Phe Pro Gly Ser Gln Lys
1               5

<210> 215
<211> 9
<212> PRT
<213> Artificial Sequence

<220>
<223> tumour differentiation antigen

<400> 215
Ala Leu Asn Phe Pro Gly Ser Gln Lys
1               5

<210> 216
<211> 9
<212> PRT
<213> Artificial Sequence

<220>
<223> tumour differentiation antigen

<400> 216
Val Tyr Phe Phe Leu Pro Asp His Leu
1               5

<210> 217
<211> 9
<212> PRT
<213> Artificial Sequence

<220>
<223> tumour differentiation antigen

<400> 217
Arg Thr Lys Gln Leu Tyr Pro Glu Trp
1               5

<210> 218
<211> 10
<212> PRT
<213> Artificial Sequence

<220>
<223> tumour differentiation antigen

<400> 218
His Thr Met Glu Val Thr Val Tyr His Arg
1               5                   10

<210> 219
<211> 9
<212> PRT
<213> Artificial Sequence

<220>
<223> tumour differentiation antigen

<400> 219
Ser Ser Pro Gly Cys Gln Pro Pro Ala
1               5

<210> 220
<211> 10
<212> PRT
<213> Artificial Sequence

<220>
<223> tumour differentiation antigen

<400> 220
Val Pro Leu Asp Cys Val Leu Tyr Arg Tyr
1               5                   10

<210> 221
<211> 9
<212> PRT
<213> Artificial Sequence

<220>
<223> tumour differentiation antigen

<400> 221
Leu Pro His Ser Ser Ser His Trp Leu
1               5

<210> 222
<211> 8
<212> PRT
<213> Artificial Sequence

<220>
<223> tumour differentiation antigen

<400> 222
Ser Asn Asp Gly Pro Thr Leu Ile
1               5

<210> 223
<211> 15
<212> PRT
<213> Artificial Sequence

<220>
<223> tumour differentiation antigen

<400> 223
Gly Arg Ala Met Leu Gly Thr His Thr Met Glu Val Thr Val Tyr
1               5                   10                  15

<210> 224
<211> 16
<212> PRT
<213> Artificial Sequence

<220>
<223> tumour differentiation antigen

<400> 224
Trp Asn Arg Gln Leu Tyr Pro Glu Trp Thr Glu Ala Gln Arg Leu Asp
1               5                   10                  15

<210> 225
<211> 18
<212> PRT
<213> Artificial Sequence

<220>
<223> tumour differentiation antigen

<400> 225
Thr Thr Glu Trp Val Glu Thr Thr Ala Arg Glu Leu Pro Ile Pro Glu
1               5                   10                  15
Pro Glu

<210> 226
<211> 17
<212> PRT
<213> Artificial Sequence

<220>
<223> tumour differentiation antigen

<400> 226
Thr Gly Arg Ala Met Leu Gly Thr His Thr Met Glu Val Thr Val Tyr
1               5                   10                  15
His

<210> 227
<211> 15
<212> PRT
<213> Artificial Sequence

<220>
<223> tumour differentiation antigen

<400> 227
Gly Arg Ala Met Leu Gly Thr His Thr Met Glu Val Thr Val Tyr
1               5                   10                  15

<210> 228
<211> 15
<212> PRT
<213> Artificial Sequence

<220>
<223> tumour differentiation antigen

<400> 228
Ser Val Ser Glu Ser Asp Thr Ile Arg Ser Ile Ser Ile Ala Ser
1               5                   10                  15

<210> 229
<211> 15
<212> PRT
<213> Artificial Sequence

<220>
<223> tumour differentiation antigen

<400> 229
Leu Leu Ala Asn Gly Arg Met Pro Thr Val Leu Gln Cys Val Asn
1               5                   10                  15

<210> 230
<211> 15
<212> PRT
<213> Artificial Sequence

<220>
<223> tumour differentiation antigen

<400> 230
Arg Met Pro Thr Val Leu Gln Cys Val Asn Val Ser Val Val Ser
1               5                   10                  15

<210> 231
<211> 9
<212> PRT
<213> Artificial Sequence

<220>
<223> tumour differentiation antigen

<400> 231
Pro Leu Leu Glu Asn Val Ile Ser Lys
1               5

<210> 232
<211> 10
<212> PRT
<213> Artificial Sequence

<220>
<223> tumour differentiation antigen

<400> 232
Glu Ala Ala Gly Ile Gly Ile Leu Thr Val
1               5                   10

<210> 233
<211> 9
<212> PRT
<213> Artificial Sequence

<220>
<223> tumour differentiation antigen

<400> 233
Ile Leu Thr Val Ile Leu Gly Val Leu
1               5

<210> 234
<211> 10
<212> PRT
<213> Artificial Sequence

<220>
<223> tumour differentiation antigen

<400> 234
Glu Ala Ala Gly Ile Gly Ile Leu Thr Val
1               5                   10

<210> 235
<211> 11
<212> PRT
<213> Artificial Sequence

<220>
<223> tumour differentiation antigen

<400> 235
Ala Glu Glu Ala Ala Gly Ile Gly Ile Leu Thr
1               5                   10

<210> 236
<211> 11
<212> PRT
<213> Artificial Sequence

<220>
<223> tumour differentiation antigen

<400> 236
Arg Asn Gly Tyr Arg Ala Leu Met Asp Lys Ser
1               5                   10

<210> 237
<211> 12
<212> PRT
<213> Artificial Sequence

<220>
<223> tumour differentiation antigen

<400> 237
Glu Glu Ala Ala Gly Ile Gly Ile Leu Thr Val Ile
1               5                   10

<210> 238
<211> 14
<212> PRT
<213> Artificial Sequence

<220>
<223> tumour differentiation antigen

<400> 238
Ala Ala Gly Ile Gly Ile Leu Thr Val Ile Leu Gly Val Leu
1               5                   10

<210> 239
<211> 13
<212> PRT
<213> Artificial Sequence

<220>
<223> tumour differentiation antigen

<400> 239
Ala Pro Pro Ala Tyr Glu Lys Leu Pro Ser Ala Glu Gln
1               5                   10

<210> 240
<211> 12
<212> PRT
<213> Artificial Sequence

<220>
<223> tumour differentiation antigen

<400> 240
Glu Glu Ala Ala Gly Ile Gly Ile Leu Thr Val Ile
1               5                   10

<210> 241
<211> 23
<212> PRT
<213> Artificial Sequence

<220>
<223> tumour differentiation antigen

<400> 241
Arg Asn Gly Tyr Arg Ala Leu Met Asp Lys Ser Leu His Val Gly Thr
1               5                   10                  15
Gln Cys Ala Leu Thr Arg Arg
            20

<210> 242
<211> 20
<212> PRT
<213> Artificial Sequence

<220>
<223> tumour differentiation antigen

<400> 242
Met Pro Arg Glu Asp Ala His Phe Ile Tyr Gly Tyr Pro Lys Lys Gly
1               5                   10                  15
His Gly His Ser
            20

<210> 243
<211> 20
<212> PRT
<213> Artificial Sequence

<220>
<223> tumour differentiation antigen

<400> 243
Lys Asn Cys Glu Pro Val Val Pro Asn Ala Pro Pro Ala Tyr Glu Lys
1               5                   10                  15
Leu Ser Ala Glu
            20

<210> 244
<211> 9
<212> PRT
<213> Artificial Sequence

<220>
<223> tumour differentiation antigen

<400> 244
Ser Leu Ser Lys Ile Leu Asp Thr Val
1               5

<210> 245
<211> 9
<212> PRT
<213> Artificial Sequence

<220>
<223> tumour differentiation antigen

<400> 245
Leu Tyr Ser Ala Cys Phe Trp Trp Leu
1               5

<210> 246
<211> 10
<212> PRT
<213> Artificial Sequence

<220>
<223> tumour differentiation antigen

<400> 246
Phe Leu Thr Pro Lys Lys Leu Gln Cys Val
1               5                   10

<210> 247
<211> 10
<212> PRT
<213> Artificial Sequence

<220>
<223> tumour differentiation antigen

<400> 247
Val Ile Ser Asn Asp Val Cys Ala Gln Val
1               5                   10

<210> 248
<211> 9
<212> PRT
<213> Artificial Sequence

<220>
<223> tumour differentiation antigen

<400> 248
Val Leu His Trp Asp Pro Glu Thr Val
1               5

<210> 249
<211> 9
<212> PRT
<213> Artificial Sequence

<220>
<223> tumour differentiation antigen

<400> 249
Met Ser Leu Gln Arg Gln Phe Leu Arg
1               5

<210> 250
<211> 21
<212> PRT
<213> Artificial Sequence

<220>
<223> tumour differentiation antigen

<400> 250
Ile Ser Pro Asn Ser Val Phe Ser Gln Trp Arg Val Val Cys Asp Ser
1               5                   10                  15
Leu Glu Asp Tyr Asp
            20

<210> 251
<211> 10
<212> PRT
<213> Artificial Sequence

<220>
<223> tumour differentiation antigen

<400> 251
Ser Leu Pro Tyr Trp Asn Phe Ala Thr Gly
1               5                   10

<210> 252
<211> 9
<212> PRT
<213> Artificial Sequence

<220>
<223> tumour differentiation antigen

<400> 252
Ser Val Tyr Asp Phe Phe Val Trp Leu
1               5

<210> 253
<211> 9
<212> PRT
<213> Artificial Sequence

<220>
<223> tumour differentiation antigen

<400> 253
Thr Leu Asp Ser Gln Val Met Ser Leu
1               5

<210> 254
<211> 9
<212> PRT
<213> Artificial Sequence

<220>
<223> tumour differentiation antigen

<400> 254
Leu Leu Gly Pro Gly Arg Pro Tyr Arg
1               5

<210> 255
<211> 9
<212> PRT
<213> Artificial Sequence

<220>
<223> tumour differentiation antigen

<400> 255
Leu Leu Gly Pro Gly Arg Pro Tyr Arg
1               5

<210> 256
<211> 9
<212> PRT
<213> Artificial Sequence

<220>
<223> tumour differentiation antigen

<400> 256
Ala Asn Asp Pro Ile Phe Val Val Leu
1               5

<210> 257
<211> 15
<212> PRT
<213> Artificial Sequence

<220>
<223> tumour differentiation antigen

<400> 257
Gln Cys Thr Glu Val Arg Ala Asp Thr Arg Pro Trp Ser Gly Pro
1               5                   10                  15

<210> 258
<211> 10
<212> PRT
<213> Artificial Sequence

<220>
<223> tumour differentiation antigen

<400> 258
Ala Leu Pro Tyr Trp Asn Phe Ala Thr Gly
1               5                   10

<210> 259
<211> 9
<212> PRT
<213> Artificial Sequence

<220>
<223> tumour differentiation antigen

<400> 259
Lys Cys Asp Ile Cys Thr Asp Glu Tyr
1               5

<210> 260
<211> 11
<212> PRT

<213> Artificial Sequence

<220>
<223> tumour differentiation antigen

<400> 260
Ser Ser Asp Tyr Val Ile Pro Ile Gly Thr Tyr
1               5                   10

<210> 261
<211> 9
<212> PRT
<213> Artificial Sequence

<220>
<223> tumour differentiation antigen

<400> 261
Met Leu Leu Ala Val Leu Tyr Cys Leu
1               5

<210> 262
<211> 10
<212> PRT
<213> Artificial Sequence

<220>
<223> tumour differentiation antigen

<400> 262
Cys Leu Leu Trp Ser Phe Gln Thr Ser Ala
1               5                   10

<210> 263
<211> 9
<212> PRT
<213> Artificial Sequence

<220>
<223> tumour differentiation antigen

<400> 263
Tyr Met Asp Gly Thr Met Ser Gln Val
1               5

<210> 264
<211> 9
<212> PRT
<213> Artificial Sequence

<220>
<223> tumour differentiation antigen

<400> 264
Ala Phe Leu Pro Trp His Arg Leu Phe
1               5

<210> 265
<211> 9
<212> PRT
<213> Artificial Sequence

<220>

<223> tumour differentiation antigen

<400> 265
Gln Cys Ser Gly Asn Phe Met Gly Phe
1               5

<210> 266
<211> 12
<212> PRT
<213> Artificial Sequence

<220>
<223> tumour differentiation antigen

<400> 266
Thr Pro Arg Leu Pro Ser Ser Ala Asp Val Glu Phe
1               5                   10

<210> 267
<211> 9
<212> PRT
<213> Artificial Sequence

<220>
<223> tumour differentiation antigen

<400> 267
Leu Pro Ser Ser Ala Asp Val Glu Phe
1               5

<210> 268
<211> 10
<212> PRT
<213> Artificial Sequence

<220>
<223> tumour differentiation antigen

<400> 268
Leu His His Ala Phe Val Asp Ser Ile Phe
1               5                   10

<210> 269
<211> 9
<212> PRT
<213> Artificial Sequence

<220>
<223> tumour differentiation antigen

<400> 269
Ser Glu Ile Trp Arg Asp Ile Asp Phe
1               5

<210> 270
<211> 15
<212> PRT
<213> Artificial Sequence

<220>
<223> tumour differentiation antigen

<400> 270

Gln Asn Ile Leu Leu Ser Asn Ala Pro Leu Gly Pro Gln Phe Pro
1               5                   10                  15

<210> 271
<211> 13
<212> PRT
<213> Artificial Sequence

<220>
<223> tumour differentiation antigen

<400> 271
Ser Tyr Leu Gln Asp Ser Asp Pro Asp Ser Phe Gln Asp
1               5                   10

<210> 272
<211> 21
<212> PRT
<213> Artificial Sequence

<220>
<223> tumour differentiation antigen

<400> 272

<210> 273
<211> 9
<212> PRT
<213> Artificial Sequence

<220>
<223> antigen overexpressed in tumour

<400> 273
Ser Val Ala Ser Thr Ile Thr Gly Val
1               5

<210> 274
<211> 10
<212> PRT
<213> Artificial Sequence

<220>
<223> antigen overexpressed in tumour

<400> 274
Arg Ser Asp Ser Gly Gln Gln Ala Arg Tyr
1               5                   10

<210> 275
<211> 9
<212> PRT
<213> Artificial Sequence

<220>
<223> antigen overexpressed in tumour

<400> 275
Leu Leu Tyr Lys Leu Ala Asp Leu Ile

1                   5

<210> 276
<211> 10
<212> PRT
<213> Artificial Sequence

<220>
<223> antigen overexpressed in tumour

<400> 276
Tyr Leu Asn Asp His Leu Glu Pro Trp Ile
1               5                   10

<210> 277
<211> 9
<212> PRT
<213> Artificial Sequence

<220>
<223> antigen overexpressed in tumour

<400> 277
Cys Gln Trp Gly Arg Leu Trp Gln Leu
1               5

<210> 278
<211> 10
<212> PRT
<213> Artificial Sequence

<220>
<223> antigen overexpressed in tumour

<400> 278
Val Leu Leu Gln Ala Gly Ser Leu His Ala
1               5                   10

<210> 279
<211> 9
<212> PRT
<213> Artificial Sequence

<220>
<223> antigen overexpressed in tumour

<400> 279
Lys Val His Pro Val Ile Trp Ser Leu
1               5

<210> 280
<211> 10
<212> PRT
<213> Artificial Sequence

<220>
<223> antigen overexpressed in tumour

<400> 280
Leu Met Leu Gln Asn Ala Leu Thr Thr Met
1               5                   10

<210> 281

<211> 9
<212> PRT
<213> Artificial Sequence

<220>
<223> antigen overexpressed in tumour

<400> 281
Leu Leu Gly Ala Thr Cys Met Phe Val
1               5

<210> 282
<211> 15
<212> PRT
<213> Artificial Sequence

<220>
<223> antigen overexpressed in tumour

<400> 282
Asn Pro Pro Ser Met Val Ala Ala Gly Ser Val Val Ala Ala Val
1               5                   10                  15

<210> 283
<211> 10
<212> PRT
<213> Artificial Sequence

<220>
<223> antigen overexpressed in tumour

<400> 283
Ala Leu Gly Gly His Pro Leu Leu Gly Val
1               5                   10

<210> 284
<211> 9
<212> PRT
<213> Artificial Sequence

<220>
<223> antigen overexpressed in tumour

<400> 284
Thr Met Asn Gly Ser Lys Ser Pro Val
1               5

<210> 285
<211> 9
<212> PRT
<213> Artificial Sequence

<220>
<223> antigen overexpressed in tumour

<400> 285
Arg Tyr Gln Leu Asp Pro Lys Phe Ile
1               5

<210> 286
<211> 12
<212> PRT
<213> Artificial Sequence

<220>
<223> antigen overexpressed in tumour

<400> 286
Asp Val Thr Phe Asn Ile Ile Cys Lys Lys Cys Gly
1               5                   10

<210> 287
<211> 9
<212> PRT
<213> Artificial Sequence

<220>
<223> antigen overexpressed in tumour

<400> 287
Phe Met Val Glu Asp Glu Thr Val Leu
1               5

<210> 288
<211> 10
<212> PRT
<213> Artificial Sequence

<220>
<223> antigen overexpressed in tumour

<400> 288
Phe Ile Asn Asp Glu Ile Phe Val Glu Leu
1               5                   10

<210> 289
<211> 9
<212> PRT
<213> Artificial Sequence

<220>
<223> antigen overexpressed in tumour

<400> 289
Lys Tyr Asp Cys Phe Leu His Pro Phe
1               5

<210> 290
<211> 9
<212> PRT
<213> Artificial Sequence

<220>
<223> antigen overexpressed in tumour

<400> 290
Lys Tyr Val Gly Ile Glu Arg Glu Met
1               5

<210> 291
<211> 9
<212> PRT
<213> Artificial Sequence

<220>
<223> antigen overexpressed in tumour

<400> 291
Asn Thr Tyr Ala Ser Pro Arg Phe Lys
1               5

<210> 292
<211> 9
<212> PRT
<213> Artificial Sequence

<220>
<223> antigen overexpressed in tumour

<400> 292
His Leu Ser Thr Ala Phe Ala Arg Val
1               5

<210> 293
<211> 9
<212> PRT
<213> Artificial Sequence

<220>
<223> antigen overexpressed in tumour

<400> 293
Lys Ile Phe Gly Ser Leu Ala Phe Leu
1               5

<210> 294
<211> 9
<212> PRT
<213> Artificial Sequence

<220>
<223> antigen overexpressed in tumour

<400> 294
Ile Ile Ser Ala Val Val Gly Ile Leu
1               5

<210> 295
<211> 9
<212> PRT
<213> Artificial Sequence

<220>
<223> antigen overexpressed in tumour

<400> 295
Ala Leu Cys Arg Trp Gly Leu Leu Leu
1               5

<210> 296
<211> 9
<212> PRT
<213> Artificial Sequence

<220>
<223> antigen overexpressed in tumour

<400> 296
Ile Leu His Asn Gly Ala Tyr Ser Leu

1                   5

<210> 297
<211> 9
<212> PRT
<213> Artificial Sequence

<220>
<223> antigen overexpressed in tumour

<400> 297
Arg Leu Leu Gln Glu Thr Glu Leu Val
1               5

<210> 298
<211> 9
<212> PRT
<213> Artificial Sequence

<220>
<223> antigen overexpressed in tumour

<400> 298
Val Val Leu Gly Val Val Phe Gly Ile
1               5

<210> 299
<211> 10
<212> PRT
<213> Artificial Sequence

<220>
<223> antigen overexpressed in tumour

<400> 299
Tyr Met Ile Met Val Lys Cys Trp Met Ile
1               5                   10

<210> 300
<211> 9
<212> PRT
<213> Artificial Sequence

<220>
<223> antigen overexpressed in tumour

<400> 300
His Leu Tyr Gln Gly Cys Gln Val Val
1               5

<210> 301
<211> 10
<212> PRT
<213> Artificial Sequence

<220>
<223> antigen overexpressed in tumour

<400> 301
Tyr Leu Val Pro Gln Gln Gly Phe Phe Cys
1               5                   10

<210> 302

<211> 9
<212> PRT
<213> Artificial Sequence

<220>
<223> antigen overexpressed in tumour

<400> 302
Pro Leu Gln Pro Glu Gln Leu Gln Val
1               5

<210> 303
<211> 9
<212> PRT
<213> Artificial Sequence

<220>
<223> antigen overexpressed in tumour

<400> 303
Thr Leu Glu Glu Ile Thr Gly Tyr Leu
1               5

<210> 304
<211> 9
<212> PRT
<213> Artificial Sequence

<220>
<223> antigen overexpressed in tumour

<400> 304
Ala Leu Ile His His Asn Thr His Leu
1               5

<210> 305
<211> 9
<212> PRT
<213> Artificial Sequence

<220>
<223> antigen overexpressed in tumour

<400> 305
Pro Leu Thr Ser Ile Ile Ser Ala Val
1               5

<210> 306
<211> 9
<212> PRT
<213> Artificial Sequence

<220>
<223> antigen overexpressed in tumour

<400> 306
Val Leu Arg Glu Asn Thr Ser Pro Lys
1               5

<210> 307
<211> 9
<212> PRT
<213> Artificial Sequence

<220>
<223> antigen overexpressed in tumour

<400> 307
Thr Tyr Leu Pro Thr Asn Ala Ser Leu
1               5

<210> 308
<211> 9
<212> PRT
<213> Artificial Sequence

<220>
<223> antigen overexpressed in tumour

<400> 308
Ala Leu Leu Glu Ile Ala Ser Cys Leu
1               5

<210> 309
<211> 9
<212> PRT
<213> Artificial Sequence

<220>
<223> antigen overexpressed in tumour

<400> 309
Trp Leu Pro Phe Gly Phe Ile Leu Ile
1               5

<210> 310
<211> 9
<212> PRT
<213> Artificial Sequence

<220>
<223> antigen overexpressed in tumour

<400> 310
Ser Pro Arg Trp Trp Pro Thr Cys Leu
1               5

<210> 311
<211> 9
<212> PRT
<213> Artificial Sequence

<220>
<223> antigen overexpressed in tumour

<400> 311
Gly Val Ala Leu Gln Thr Met Lys Gln
1               5

<210> 312
<211> 9
<212> PRT
<213> Artificial Sequence

<220>
<223> antigen overexpressed in tumour

<400> 312
Phe Met Asn Lys Phe Ile Tyr Glu Ile
1               5

<210> 313
<211> 10
<212> PRT
<213> Artificial Sequence

<220>
<223> antigen overexpressed in tumour

<400> 313
Gln Leu Ala Val Ser Val Ile Leu Arg Val
1               5                   10

<210> 314
<211> 14
<212> PRT
<213> Artificial Sequence

<220>
<223> antigen overexpressed in tumour

<400> 314
Leu Pro Ala Val Val Gly Leu Ser Pro Gly Glu Gln Glu Tyr
1               5                   10

<210> 315
<211> 16
<212> PRT
<213> Artificial Sequence

<220>
<223> antigen overexpressed in tumour

<400> 315
Val Gly Gln Asp Val Ser Val Leu Phe Arg Val Thr Gly Ala Leu Gln
1               5                   10                  15

<210> 316
<211> 8
<212> PRT
<213> Artificial Sequence

<220>
<223> antigen overexpressed in tumour

<400> 316
Val Leu Phe Tyr Leu Gly Gln Tyr
1               5

<210> 317
<211> 9
<212> PRT
<213> Artificial Sequence

<220>
<223> antigen overexpressed in tumour

<400> 317
Thr Leu Asn Asp Glu Cys Trp Pro Ala
1               5

<210> 318
<211> 9
<212> PRT
<213> Artificial Sequence

<220>
<223> antigen overexpressed in tumour

<400> 318
Gly Leu Pro Pro Asp Val Gln Arg Val
1               5

<210> 319
<211> 12
<212> PRT
<213> Artificial Sequence

<220>
<223> antigen overexpressed in tumour

<400> 319
Ser Leu Phe Pro Asn Ser Pro Lys Trp Thr Ser Lys
1               5                   10

<210> 320
<211> 9
<212> PRT
<213> Artificial Sequence

<220>
<223> antigen overexpressed in tumour

<400> 320
Ser Thr Ala Pro Pro Val His Asn Val
1               5

<210> 321
<211> 9
<212> PRT
<213> Artificial Sequence

<220>
<223> antigen overexpressed in tumour

<400> 321
Leu Leu Leu Leu Thr Val Leu Thr Val
1               5

<210> 322
<211> 12
<212> PRT
<213> Artificial Sequence

<220>
<223> antigen overexpressed in tumour

<400> 322
Pro Gly Ser Thr Ala Pro Pro Ala His Gly Val Thr
1               5                   10

<210> 323
<211> 9
<212> PRT
<213> Artificial Sequence

<220>
<223> antigen overexpressed in tumour

<400> 323
Leu Leu Gly Arg Asn Ser Phe Glu Val
1               5

<210> 324
<211> 9
<212> PRT
<213> Artificial Sequence

<220>
<223> antigen overexpressed in tumour

<400> 324
Arg Met Pro Glu Ala Ala Pro Pro Val
1               5

<210> 325
<211> 9
<212> PRT
<213> Artificial Sequence

<220>
<223> antigen overexpressed in tumour

<400> 325
Ser Gln Lys Thr Tyr Gln Gly Ser Tyr
1               5

<210> 326
<211> 13
<212> PRT
<213> Artificial Sequence

<220>
<223> antigen overexpressed in tumour

<400> 326
Pro Gly Thr Arg Val Arg Ala Met Ala Ile Tyr Lys Gln
1               5                   10

<210> 327
<211> 12
<212> PRT
<213> Artificial Sequence

<220>
<223> antigen overexpressed in tumour

<400> 327
His Leu Ile Arg Val Glu Gly Asn Leu Arg Val Glu
1               5                   10

<210> 328
<211> 9

<212> PRT
<213> Artificial Sequence

<220>
<223> antigen overexpressed in tumour

<400> 328
Thr Leu Pro Gly Tyr Pro Pro His Val
1               5

<210> 329
<211> 12
<212> PRT
<213> Artificial Sequence

<220>
<223> antigen overexpressed in tumour

<400> 329
Cys Thr Ala Cys Arg Trp Lys Lys Ala Cys Gln Arg
1               5                   10

<210> 330
<211> 9
<212> PRT
<213> Artificial Sequence

<220>
<223> antigen overexpressed in tumour

<400> 330
Val Leu Asp Gly Leu Asp Val Leu Leu
1               5

<210> 331
<211> 10
<212> PRT
<213> Artificial Sequence

<220>
<223> antigen overexpressed in tumour

<400> 331
Ser Leu Tyr Ser Phe Pro Glu Pro Glu Ala
1               5                   10

<210> 332
<211> 10
<212> PRT
<213> Artificial Sequence

<220>
<223> antigen overexpressed in tumour

<400> 332
Ala Leu Tyr Val Asp Ser Leu Phe Phe Leu
1               5                   10

<210> 333
<211> 9
<212> PRT
<213> Artificial Sequence

<220>
<223> antigen overexpressed in tumour

<400> 333
Ser Leu Leu Gln His Leu Ile Gly Leu
1               5

<210> 334
<211> 9
<212> PRT
<213> Artificial Sequence

<220>
<223> antigen overexpressed in tumour

<400> 334
Leu Tyr Val Asp Ser Leu Phe Phe Leu
1               5

<210> 335
<211> 9
<212> PRT
<213> Artificial Sequence

<220>
<223> antigen overexpressed in tumour

<400> 335
Asn Tyr Ala Arg Thr Glu Asp Phe Phe
1               5

<210> 336
<211> 9
<212> PRT
<213> Artificial Sequence

<220>
<223> antigen overexpressed in tumour

<400> 336
Leu Lys Leu Ser Gly Val Val Arg Leu
1               5

<210> 337
<211> 10
<212> PRT
<213> Artificial Sequence

<220>
<223> antigen overexpressed in tumour

<400> 337
Pro Leu Pro Pro Ala Arg Asn Gly Gly Leu
1               5                   10

<210> 338
<211> 10
<212> PRT
<213> Artificial Sequence

<220>
<223> antigen overexpressed in tumour

<400> 338
Ser Pro Ser Ser Asn Arg Ile Arg Asn Thr
1               5                   10

<210> 339
<211> 9
<212> PRT
<213> Artificial Sequence

<220>
<223> antigen overexpressed in tumour

<400> 339
Leu Ala Ala Leu Pro His Ser Cys Leu
1               5

<210> 340
<211> 10
<212> PRT
<213> Artificial Sequence

<220>
<223> antigen overexpressed in tumour

<400> 340
Gly Leu Ala Ser Phe Lys Ser Phe Leu Lys
1               5                   10

<210> 341
<211> 10
<212> PRT
<213> Artificial Sequence

<220>
<223> antigen overexpressed in tumour

<400> 341
Arg Ala Gly Leu Gln Val Arg Lys Asn Lys
1               5                   10

<210> 342
<211> 10
<212> PRT
<213> Artificial Sequence

<220>
<223> antigen overexpressed in tumour

<400> 342
Ala Leu Trp Pro Trp Leu Leu Met Ala Thr
1               5                   10

<210> 343
<211> 9
<212> PRT
<213> Artificial Sequence

<220>
<223> antigen overexpressed in tumour

<400> 343
Asn Ser Gln Pro Val Trp Leu Cys Leu
1               5

<210> 344
<211> 9
<212> PRT
<213> Artificial Sequence

<220>
<223> antigen overexpressed in tumour

<400> 344
Leu Pro Arg Trp Pro Pro Pro Gln Leu
1               5

<210> 345
<211> 9
<212> PRT
<213> Artificial Sequence

<220>
<223> antigen overexpressed in tumour

<400> 345
Lys Met Asp Ala Glu His Pro Glu Leu
1               5

<210> 346
<211> 9
<212> PRT
<213> Artificial Sequence

<220>
<223> antigen overexpressed in tumour

<400> 346
Ala Trp Ile Ser Lys Pro Pro Gly Val
1               5

<210> 347
<211> 10
<212> PRT
<213> Artificial Sequence

<220>
<223> antigen overexpressed in tumour

<400> 347
Ser Ala Trp Ile Ser Lys Pro Pro Gly Val
1               5                   10

<210> 348
<211> 9
<212> PRT
<213> Artificial Sequence

<220>
<223> antigen overexpressed in tumour

<400> 348
Met Ile Ala Val Phe Leu Pro Ile Val
1               5

<210> 349
<211> 15

<212> PRT
<213> Artificial Sequence

<220>
<223> antigen overexpressed in tumour

<400> 349
His Gln Gln Tyr Phe Tyr Lys Ile Pro Ile Leu Val Ile Asn Lys
1               5                   10                  15

<210> 350
<211> 10
<212> PRT
<213> Artificial Sequence

<220>
<223> antigen overexpressed in tumour

<400> 350
Glu Leu Thr Leu Gly Glu Phe Leu Lys Leu
1               5                   10

<210> 351
<211> 9
<212> PRT
<213> Artificial Sequence

<220>
<223> antigen overexpressed in tumour

<400> 351
Ile Leu Ala Lys Phe Leu His Trp Leu
1               5

<210> 352
<211> 9
<212> PRT
<213> Artificial Sequence

<220>
<223> antigen overexpressed in tumour

<400> 352
Arg Leu Val Asp Asp Phe Leu Leu Val
1               5

<210> 353
<211> 15
<212> PRT
<213> Artificial Sequence

<220>
<223> antigen overexpressed in tumour

<400> 353
Arg Pro Gly Leu Leu Gly Ala Ser Val Leu Gly Leu Asp Asp Ile
1               5                   10                  15

<210> 354
<211> 15
<212> PRT
<213> Artificial Sequence

<220>
<223> antigen overexpressed in tumour

<400> 354
Leu Thr Asp Leu Gln Pro Tyr Met Arg Gln Phe Val Ala His Leu
1               5                   10                  15

<210> 355
<211> 9
<212> PRT
<213> Artificial Sequence

<220>
<223> antigen overexpressed in tumour

<400> 355
Ser Arg Phe Gly Gly Ala Val Val Arg
1               5

<210> 356
<211> 11
<212> PRT
<213> Artificial Sequence

<220>
<223> antigen overexpressed in tumour

<400> 356
Thr Ser Glu Lys Arg Pro Phe Met Cys Ala Tyr
1               5                   10

<210> 357
<211> 9
<212> PRT
<213> Artificial Sequence

<220>
<223> antigen overexpressed in tumour

<400> 357
Cys Met Thr Trp Asn Gln Met Asn Leu
1               5

<210> 358
<211> 11
<212> PRT
<213> Artificial Sequence

<220>
<223> antigen overexpressed in tumour

<400> 358
Leu Ser His Leu Gln Met His Ser Arg Lys His
1               5                   10

<210> 359
<211> 16
<212> PRT
<213> Artificial Sequence

<220>
<223> antigen overexpressed in tumour

<400> 359
Lys Arg Tyr Phe Lys Leu Ser His Leu Gln Met His Ser Arg Lys His
1               5                   10                  15

<210> 360
<211> 10
<212> PRT
<213> Artificial Sequence

<220>
<223> tumour antigen

<220>
<221> CHAIN
<222> 1..10
<223> Ndufs1

<400> 360
Ala Ala Val Ser Asn Met Val Gln Lys Ile
1               5                   10